# EUROPÄISCHE PATENTSCHRIFT

(11) **EP 2 320 854 B1**
(45) Veröffentlichungstag und Bekanntmachung des Hinweises auf die Patenterteilung: **18.01.2012**
(21) Anmeldenummer: 09782550.9
(22) Anmeldetag: 03.09.2009
(51) Int. Cl.: A61K 8/46, A61K 8/49, A61K 8/22, A61Q 5/08, A61K 8/86

(54) **AUFHELLMITTEL MIT 2-ACYLPYRIDINIUM-DERIVATEN**
BLEACHING AGENT COMPRISING 2-ACYLPYRIDINIUM DERIVATIVES
AGENT ÉCLAIRCISSANT COMPRENANT DES DÉRIVÉS DE 2-ACYLPYRIDINIUM

(30) Priorität: 09.09.2008 DE 102008046433
(43) Veröffentlichungstag der Anmeldung: 18.05.2011
(73) Patentinhaber: Henkel AG & Co. KGaA, 40589 Düsseldorf (DE)
(72) Erfinder: GROSS, Wibke, 41836 Hückelhoven (DE); FUHR, Denise, 22559 Hamburg (DE); NEMITZ, Ralph, 41363 Jüchen (DE)
(86) Internationale Anmeldenummer: PCT/EP2009/061389
(87) Internationale Veröffentlichungsnummer: WO 2010/029006

(56) Entgegenhaltungen:
- EP-A2- 1 882 495
- EP-A2- 1 905 418
- WO-A2-2010/054981
- DE-A1- 10 148 845
- DE-A1- 10 261 656
- DE-A1- 19 745 356
- DE-A1-102007 047 685

## Beschreibung

Die vorliegende Erfindung betrifft Mittel zum Aufhellen keratinischer Fasern, d.h. Mittel zur Anwendung auf Keratinfasern, insbesondere menschlichen Haaren, enthaltend kationische 2-Acylpyridinium-Derivate, ein anionisches Tensid, ausgewählt aus Alkylethersulfaten, und Wasserstoffperoxid zum Aufhellen von Haaren sowie deren Verwendung zum Aufhellen keratinischer Fasern und ein entsprechendes Verfahren.

Die Veränderung von Form und Farbe der Haare stellt einen wichtigen Bereich der modernen Kosmetik dar. Dadurch kann das Erscheinungsbild der Haare sowohl aktuellen Modeströmungen als auch den individuellen Wünschen der einzelnen Person angepasst werden. Dabei können Dauerwell- und andere die Haarform verändernde Verfahren nahezu unabhängig vom Typ der zu behandelnden Haare eingesetzt werden. Dagegen sind Färbe- und Blondierverfahren auf bestimmte Ausgangshaarfarben begrenzt. Die Grundlagen der Blondierverfahren sind dem Fachmann bekannt und können in einschlägigen Monographien, z. B. von Kh. Schrader, Grundlagen und Rezepturen der Kosmetika, 2. Auflage, 1989, Dr. Alfred Hüthig Verlag, Heidelberg, oder W. Umbach (Hrg.), Kosmetik, 2. Auflage, 1995, Georg Thieme Verlag, Stuttgart, New York, nachgelesen werden.

Neben der Färbung ist das Aufhellen der eigenen Haarfarbe bzw. das Blondieren der ganz spezielle Wunsch vieler Verbraucher, da eine blonde Haarfarbe als attraktiv und in modischer Hinsicht erstrebenswert betrachtet wird. Für diesen Zweck sind im Markt verschiedene Blondiermittel mit unterschiedlicher Blondierleistung erhältlich. Die in diesen Produkten enthaltenen Oxidationsmittel sind in der Lage, durch die oxidative Zerstörung des haareigenen Farbstoffes Melanin die Haarfaser aufzuhellen. Für einen moderaten Blondiereffekt genügt der Einsatz von Wasserstoffperoxid - gegebenenfalls unter Einsatz von Ammoniak oder anderen Alkalisierungsmitteln - als Oxidationsmittel allein, für das Erzielen eines stärkeren Blondiereffektes wird üblicherweise eine Mischung aus Wasserstoffperoxid und Peroxodisulfatsalzen und/oder Peroxomonosulfatsalzen eingesetzt. Mit der Aufhellung geht jedoch auch eine Schädigung des Haares einher, da nicht nur die natürlichen farbgebenden Komponenten des Haares, sondern auch die übrigen Strukturbestandteile des Haares oxidativ geschädigt werden. Je nach Ausprägung des Schädigungsgrades reicht dieser von rauem, sprödem und schwieriger auskämmbarem Haar über eine verminderte Widerstandsfähigkeit und Reißfestigkeit des Haares bis hin zu Haarbruch. Je größer die Menge des eingesetzten Wasserstoffperoxids und gegebenenfalls der Peroxodisulfate ist, desto stärkere Schädigungen werden in der Regel auf der Keratinfaser hervorgerufen. Haarfärbe- bzw. Aufhellmittel, welche eine gute Aufhellleistung zeigen, ohne gleichzeitig die Haarfaser zu schädigen, sind bislang nicht bekannt.

Vor ihrer Anwendung auf menschliches Haar werden Aufhellmittel in fester oder pastöser Form üblicherweise mit verdünnter wässriger Wasserstoffperoxid-Lösung vermischt. Diese Mischung wird dann auf das Haar aufgebracht und nach einer bestimmten Einwirkzeit wieder ausgespült. Die Einwirkungsdauer auf dem Haar zur Erzielung einer vollständigen Aufhellung liegt zwischen etwa 30 und 40 Minuten. Es ist nahe liegend, dass bei den Benutzern dieser Blondiermittel ein Bedürfnis besteht, diese Einwirkungszeit zu verringern.

Blondierprozesse an keratinischen Fasern laufen üblicherweise bei alkalischen pH-Werten ab, insbesondere zwischen 9,0 und 10,5. Diese pH-Werte sind notwendig, um eine Öffnung der äußeren Schuppenschicht (Cuticula) zu gewährleisten und eine Penetration der aktiven Spezies (Farbstoffvorprodukte und/oder Wasserstoffperoxid) in das Haar zu ermöglichen. Als Alkalisierungsmittel wird üblicherweise Ammoniak eingesetzt, der allerdings für den Anwender den Nachteil des intensiven Geruches und eventueller Reizung bis hin zu Hautirritationen und Hautsensibilisierungen aufweist.

Auch wenn die bislang auf dem Markt befindlichen Blondiermittel in der Regel gute Aufhellleistungen zeigen, so können sie aufgrund von Haarschädigung, langen Anwendungszeiten und der aufgrund der hohen Konzentrationen an Oxidations- und Alkalisierungsmittel möglichen Hautreizungen nicht als optimal angesehen werden.

Der Einsatz von kationischen Acylpyridinium-Derivaten in der Haarfärbung ist beispielsweise aus den Schriften DE 10148845 A1 oder DE 10261656 A1 bekannt. In beiden Dokumenten werden diese Derivate jedoch zusammen mit mindestens einer zweiten färbenden Komponente als Mittel zur Färbung und damit zur Steigerung der Farbintensität des Haares beschrieben DE 10 2007 047 685 offenbart Aufhellmittel mit kadionischen Acylpyridinium derivaten, midazdderivaten und wasserstoffperoxid. Aus dem Stand der Technik ist bislang nicht ersichtlich, dass diese 2-Acylpyridinium-Derivate in spezieller Kombination mit anionischen Tensiden vom Alkylethersulfat-Typ und Wasserstoffperoxid für die Bleiche des Haares mit sehr guter Entfärbekraft eingesetzt werden können.

Es ist die Aufgabe dieser Erfindung, neuartige Mittel zum Aufhellen bzw. Blondieren von Haaren bereitzustellen, welche in ihrer Aufhellleistung den üblichen auf dem Markt befindlichen Mitteln vergleichbar oder überlegen sind, gleichzeitig jedoch eine verringerte Haarschädigung aufweisen.

In nicht vorhersehbarer Weise konnte nun gefunden werden, dass der Einsatz einer Kombination von kationischen 2-Acylpyridiniumverbindungen der allgemeinen Struktur (I), mindestens einem anionischen Tensid, ausgewählt aus Fettalkoholethersulfaten, und Wasserstoffperoxid die Haare viel stärker aufhellt, als es durch den Einsatz einer vergleichbaren Menge Wasserstoffperoxid allein möglich wäre.

Bedingt durch die verbesserte Blondierleistung bei Anwendung des erfindungsgemäßen Mittels kann die Menge an eingesetztem Oxidationsmittel vermindert und die Haarschädigung hierdurch minimiert werden. Auch eine Verkürzung der Einwirkzeit unter Erzielung eines dem Stand der Technik entsprechenden Aufhelleffekts ist auf diese Weise möglich.

Die erfindungsgemäßen Mittel entfärben den natürlichen Farbstoff Melanin durch Oxidation. Die erfindungsgemäße Wirkstoffkombination bildet ohne die Gegenwart zusätzlicher Farbstoffe/Farbstoffvorprodukte sichtbar keinen Farbstoff in der keratinhaltigen Faser aus. Auch zuvor auf beziehungsweise in der keratinhaltigen Faser befindliche synthetische Farbstoffe können mit Hilfe der erfindungsgemäßen Mittel ausgeblichen werden.

Ein erster Gegenstand der Erfindung ist daher ein Mittel zum Aufhellen von keratinischen Fasern,
dadurch gekennzeichnet, dass es in einem kosmetischen Träger
(i) mindestens ein kationisches 2-Acylpyridinium-Derivat der Formel (I), 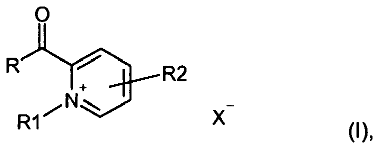 worin
   - R: für eine C₁-C₄-Alkylgruppe, eine C₂-C₆-Hydroxyalkylgruppe oder eine C₁-C₆-Alkoxy-C₂-C₆-alkylgruppe,
   - R1: für eine C₁-C₆-Alkylgruppe, eine C₂-C₆-Alkenylgruppe, eine C₂-C₆-Hydroxyalkylgruppe, eine C₁-C₆-Alkoxy-C₂-C₆-alkylgruppe, eine Carboxy-C₁-C₆-alkylgruppe, eine Aryl-C₁-C₆-alkylgruppe, eine Heteroaryl-C₁-C₆-alkylgruppe, eine Mono- oder Di-(C₁-C₆-alkyl)amino-C₂-C₆-alkylgruppe, eine 3-Oxobutylgruppe, eine 2-Oxopropylgruppe, eine Arylgruppe oder eine Heteroarylgruppe steht,
   - R2: für Wasserstoff, ein Halogen, eine Nitrilgruppe, eine Nitrogruppe, eine Aminogruppe, eine Mono- oder Di-(C₁-C₆-alkyl)aminogruppe, eine Hydroxygruppe, eine C₁-C₆-Alkoxygruppe, eine C₁-C₆-Alkylgruppe, eine partiell oder vollständig halogenierte C₁-C₆-Alkylgruppe, eine C₂-C₆-Alkenylgruppe, eine C₂-C₆-Hydroxyalkylgruppe, C₁-C₆-Alkoxy-C₂-C₆-alkylgruppe, eine C₁-C₆-Alkoxycarbonylgruppe, eine Carboxygruppe, eine Carbamoylgruppe oder eine Acylgruppe R'C(O) steht, worin R' für C₁-C₄-Alkylgruppe, eine C₂-C₆-Hydroxyalkylgruppe oder eine C₁-C₆-Alkoxy-C_{Z}-C₆-alkylgruppe steht,
   - X⁻: für ein physiologisch verträgliches Anion steht,
(ii) mindestens ein anionisches Tensid, ausgewählt aus Alkylethersulfaten gemäß der Formel R"-(OC₂H₄)ₙ-OSO₃M,
   worin R" für eine lineare oder verzweigte, gesättigte oder gegebenenfalls ungesättigte C₈-C₃₀-Alkylkette, n für eine Zahl größer als 2 und M für Wasserstoff oder ein physiologisch verträgliches Kation steht,
   und
(iii) Wasserstoffperoxid oder eine feste Anlagerungsverbindung von Wasserstoffperoxid an anorganische oder organische Verbindungen
   enthält.

Unter keratinischen Fasern oder auch Keratinfasern sind dabei Pelze, Wolle, Federn und insbesondere menschliche Haare zu verstehen. Obwohl die erfindungsgemäßen Mittel in erster Linie zum Aufhellen von Keratinfasern geeignet sind, steht prinzipiell einer Verwendung auch auf anderen Gebieten nichts entgegen.

Die erfindungsgemäßen Mittel enthalten die Wirkstoffe in einem kosmetischen Träger. Dieser kosmetische Träger ist bevorzugt wässrig, alkoholisch oder wässrig-alkoholisch. Zum Zwecke der Haarbleiche sind solche Träger beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind. Es ist aber auch möglich, zur Lagerung eine pulverförmige oder auch tablettenförmige Formulierung bereitzustellen, was für Aufhellmittel bevorzugt ist. Diese wird dann vor der Anwendung in einem wässrigen Lösungsmittel oder mit organischen Lösungsmitteln bzw. mit Gemischen aus Wasser und organischen Lösungsmitteln unter Erhalt der Anwendungsmischung vermengt. Ein wässriger Träger enthält im Sinne der Erfindung mindestens 40 Gew.-%, insbesondere mindestens 50 Gew.-% Wasser. Unter wässrig-alkoholischen Trägern sind im Sinne der vorliegenden Erfindung wasserhaltige Zusammensetzungen, enthaltend 3 bis 70 Gew.-% eines C₁-C₄-Alkohols, insbesondere Ethanol bzw. Isopropanol, zu verstehen. Die erfindungsgemäßen Mittel können zusätzlich weitere organische Lösungsmittel, wie beispielsweise 4-Methoxybutanol, Ethyldiglykol, 1,2-Propylenglykol, n-Propanol, n-Butanol, n-Butylenglykol, Glycerin, Diethylenglykolmonoethylether, und Diethylenglykolmono-n-butylether, enthalten. Bevorzugt sind dabei alle wasserlöslichen organischen Lösungsmittel. Bevorzugte erfindungsgemäße Mittel sind dadurch gekennzeichnet, dass sie zusätzlich ein nichtwässriges Lösungsmittel enthalten, wobei besonders bevorzugte erfindungsgemäße Mittel das Lösungsmittel in einer Konzentration von 0,1 bis 30 Gew.-%, bevorzugt in einer Konzentration von 1 bis 20 Gew.-%, ganz besonders bevorzugt in einer Konzentration von 2 bis 10 Gew.-%, jeweils bezogen auf das Mittel, enthalten.

Im Folgenden werden Beispiele für die als Substituenten der Verbindungen der Formel (I) genannten Reste aufgezählt:
Beispiele für C₁-C₄-Alkylreste sind die Gruppen -CH₃, -CH₂CH₃, -CH₂CH₂CH₃, -CH(CH₃)₂, -CH₂CH₂CH₂CH₃, -CH₂CH(CH₃)₂, -CH(CH₃)CH₂CH₃, -C(CH₃)₃.
Beispiele für C₁-C₆-Alkylreste sind die Gruppen -CH₃, -CH₂CH₃, -CH₂CH₂CH₃, -CH(CH₃)₂, -CH₂CH₂CH₂CH₃, -CH₂CH(CH₃)₂, -CH(CH₃)CH₂CH₃, -C(CH₃)₃, -(CH₂)₄CH₃, -(CH₂)₅CH₃.
Besonders bevorzugte Alkylreste sind Methyl und Ethyl.
Beispiele für partiell oder vollständig halogenierte C₁-C₆-Alkylreste sind die Gruppen -CH₂F, -CH₂Cl, -CF₃, -CH₂CF₃, -CF₂CF₃ oder -CH(CF₃)₂, insbesondere -CF₃.
Beispiele für eine C₂-C₆-Alkenylgruppe sind eine Prop-2-enylgruppe (Allylgruppe), eine 2-Methylprop-2-enylgruppe, eine But-3-enylgruppe, eine But-2-enylgruppe, eine Pent-4-enylgruppe oder eine Pent-3-enylgruppe. Die Prop-2-enylgruppe ist in diesem Zusammenhang besonders bevorzugt.
Beispiele für eine C₁-C₆-Alkoxygruppe sind -OCH₃, -OCH₂CH₃, -OCH₂CH₂CH₃, -OCH(CH₃)₂ oder - OC(CH₃)₃, wobei die Methoxygruppe (-OCH₃) bevorzugt ist.
Beispiele für eine C₁-C₆-Alkoxycarbonylgruppe sind -C(O)OCH₃, -C(O)OCH₂CH₃, -C(O)OCH₂CH₂CH₃, -C(O)OCH(CH₃)₂ oder -C(O)OC(CH₃)₃, wobei die Methoxycarbonylgruppe (-C(O)OCH₃) bevorzugt ist.
Beispiele für ein Halogen sind Fluor, Chlor, Brom oder Iod, insbesondere Fluor und Chlor. Weiterhin können als bevorzugte Beispiele für eine C₂-C₆-Hydroxyalkylgruppe -CH₂CH₂OH, -CH₂CH₂CH₂OH, -CH₂CH(OH)CH₃, -CH₂CH₂CH₂CH₂OH eingesetzt werden, wobei die Gruppe -CH₂CH₂OH bevorzugt ist.
Beispiele für C₁-C₆-Alkoxy-C₂-C₆-alkylgruppen sind die Gruppen -CH₂CH₂OCH₃, -CH₂CH₂CH₂OCH₃, -CH₂CH₂OCH₂CH₃, -CH₂CH₂CH₂OCH₂CH₃, -CH₂CH₂OCH(CH₃)₂, -CH₂CH₂CH₂OCH(CH₃)₂.
Beispiele für eine Carboxy-C₁-C₆-alkylgruppe sind die Carboxymethylgruppe, die 2-Carboxyethylgruppe oder die 3-Carboxypropylgruppe.
Beispiele für Aryl-C₁-C₆-alkylgruppen sind die Benzylgruppe und die 2-Phenylethylgruppe.
Beispiele für eine Heteroaryl-C₁-C₆-alkylgruppe sind die Pyridin-2-ylmethylgruppe, die Pyridin-3-ylmethylgruppe, die Pyridin-4-ylmethylgruppe, die Pyrimidin-2-ylmethylgruppe, die Pyrrol-1-yl-methylgruppe, die Pyrrol-1-ylethylgruppe, die Pyrazol-1-ylmethylgruppe oder die Pyrazol-1-yl-ethylgruppe.
Beispiele für eine Mono- oder Di-(C₁-C₆-alkyl)aminO-C₂-C₆-alkylgruppe sind die 2-Methylaminoethylgruppe, 2-Ethylaminoethylgruppe, 2-Dimethylaminoethylgruppe, 2-Diethylaminoethylgruppe, 3-Methylaminopropylgruppe, 3-Dimethylaminopropylgruppe, 2-(Piperidin-1-yl)ethylgruppe, 2-(Pyrrolidin-1-yl)ethylgruppe, 2-(Morpholin-1-yl)ethylgruppe und 2-Bis-(2-hydroxyethyl)aminoethylgruppe wobei die 2-Dimethylaminoethylgruppe und die 2-Diethylaminoethyl-gruppe besonders bevorzugt sind.
Ein Beispiel für eine Arylgruppe ist die Phenylgruppe, die 1-Naphthylgruppe oder die 2-Naphthylgruppe.
Beispiele für eine Heteroarylgruppe sind die Pyridin-2-ylgruppe, die Pyridin-3-ylgruppe, die Pyridin-4-ylgruppe, die Pyrimidin-2-ylgruppe, die Pyrrol-1-ylgruppe, die Pyrrol-2-ylgruppe, die Pyrazol-1-ylgruppe, die Pyrazol-3-ylgruppe oder die Pyrazol-2-ylgruppe.

Die erfindungsgemäßen Mittel enthalten mindestens drei wesentliche Bestandteile: mindestens ein kationisches 2-Acylpyridinium-Derivat der Formel (I), mindestens ein anionisches Tensid der Fettalkoholethersulfate und Wasserstoffperoxid. Erfindungsgemäße Mittel können auch "Anwendungsmischungen" sein, d.h. Mittel, die zwar (z.B. aus Stabilitätsgründen) getrennt verpackt vorliegen, vor der Anwendung aber miteinander zu einer Anwendungsmischung vermischt und dann appliziert werden.

Es eignen sich solche Verbindungen gemäß Formel (I) bevorzugt, bei welchem der Rest R1 der allgemeinen Struktur (I) für eine C₁-C₆-Alkylgruppe, für eine C₂-C₆-Alkenylgruppe oder für eine C₂-C₆-Hydroxyalkylgruppe steht. Insbesondere ist es bevorzugt, wenn der Rest R1 der Formel (I) für eine C₁-C₆-Alkylgruppe, besonders bevorzugt für Methyl, steht.

Es ist erfindungsgemäß weiterhin bevorzugt, wenn der Rest R2 in der Formel (I) für Wasserstoff, eine C₁-C₆-Alkylgruppe oder eine Acylgruppe R'C(O) steht, worin R' für C₁-C₄-Alkylgruppe steht. Besonders bevorzugt steht R2 für Wasserstoff oder eine Acetylgruppe, das heisst R' der Acylgruppe R'C(O) steht für Methyl.

Es ist bevorzugt, wenn das Anion X⁻ gemäß Formel (I) ausgewählt wird aus Halogenid, insbesondere Chlorid, Bromid und Iodid, Benzolsulfonat, p-Toluolsulfonat, C₁-C₄-Alkylsulfonat, Trifluormethansulfonat, Acetat, Trifluoracetat, Perchlorat, ½ Sulfat, Hydrogensulfat, Tetrafluorborat, Hexafluorphosphat oder Tetrachlorzinkat. Efindungsgemäß besonders begünstigt ist es, wenn das physiologisch verträgliche Anion X⁻ für ein Halogenidion (insbesondere Chlorid oder Bromid), Hydrogensulfat, ½ Sulfat, p-Toluolsulfonat, Benzolsulfonat oder Acetat steht.

Besonders bevorzugte kationische 2-Acylpyridinium-Derivate der allgemeinen Formel (I) sind

| | | |
|---|---|---|
| 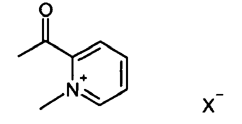 | 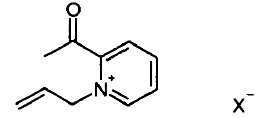 | 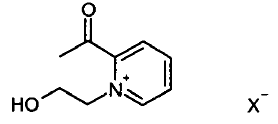 |
| Salze des 2-Acetyl-1-methylpyridiniums | Salze des 2-Acetyl-1-allylpyridiniums | Salze des 2-Acetyl-1-(2-hydroxyethyl)pyridiniums |
| 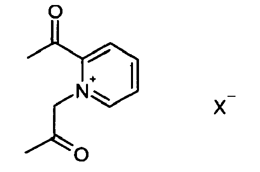 | 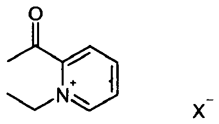 | 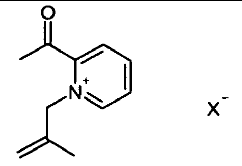 |
| Salze des 2-Acetyl-1-(2-oxopropyl)pyridiniums | Salze des 2-Acetyl-1-ethylpyridiniums | Salze des 2-Acetyl-1-(2-methyl-prop-2-enyl)-pyridiniums |
| 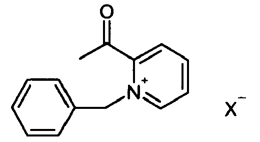 | 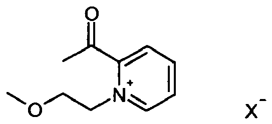 | 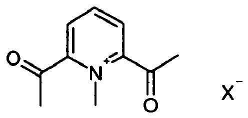 |
| Salze des 2-Acetyl-1-benzylpyridiniums | Salze des 2-Acetyl-1-(2-methoxyethyl)pyridiniums | Salze des 2,6-Diacetyl-1-methylpyridiniums |
| 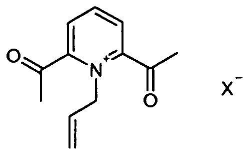 | 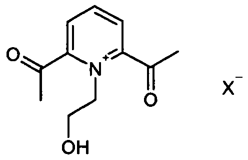 | 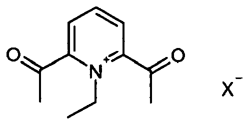 |
| Salze des 2,6-Diacetyl-1-allylpyridiniums | Salze des 2,6-Diacetyl-1-(2-hydroxyethyl)pyridiniums | Salze des 2,6-Diacetyl-1-ethylpyridiniums |
| 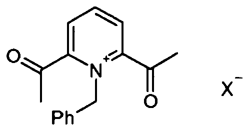 | 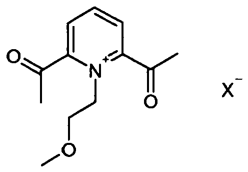 | 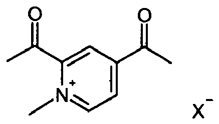 |
| Salze des 2,6-Diacetyl-1-benzylpyridiniums | Salze des 2,6-Diacetyl-1-(2-methoxyethyl)pyridiniu ms | Salze des 2,4-Diacetyl-1-methylpyridiniums |
| 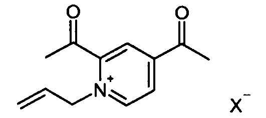 | 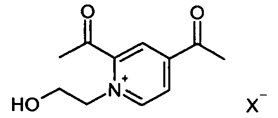 | 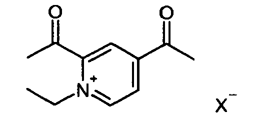 |
| Salze des 2,4-Diacetyl-1-allylpyridiniums | Salze des 2,4-Diacetyl-1-(2-hydroxyethyl)pyridiniums | Salze des 2,4-Diacetyl-1-ethylpyridiniums |
| 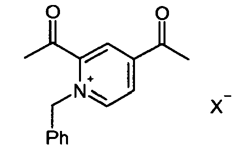 | 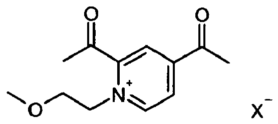 | |
| Salze des 2,4-Diacetyl-1-benzylpyridiniums | Salze des 2,4-Diacetyl-1-(2-methoxyethyl)pyridiniums | |

worin X⁻ jeweils die Bedeutungen gemäß Struktur (I) bzw. die Bedeutung der vorgenannten bevorzugten Ausführungsformen annimmt.

Zusammenfassend sind erfindungsgemäße Mittel bevorzugt, die als kationisches 2-Acylpyridinium-Derivat der allgemeinen Struktur (I) mindestens eine Verbindung aus der Gruppe enthalten, die gebildet wird aus 2-Acetyl-1-methylpyridinium-p-toluolsulfonat, 2-Acetyl-1-methylpyridiniumbenzolsulfonat, 2-Acetyl-1-methylpyridiniumbromid, 2-Acetyl-1-methylpyridiniumchlorid, 2-Acetyl-1-methylpyridiniumhydrogensulfat, 2-Acetyl-1-methylpyridiniumacetat, 2-Acetyl-1-allylpyridiniump-toluosulfonat, 2-Acetyl-1-allylpyridiniumbenzolsulfonat, 2-Acetyl-1-allylpyridiniumbromid, 2-Acetyl-1-allylpyridiniumchlorid, 2-Acetyl-1-allylpyridiniumhydrogensulfat, 2-Acetyl-1-allylpyridiniumacetat, 2-Acetyl-1-(2-hydroxyethyl)pyridinium-p-toluolsulfonat, 2-Acetyl-1-(2-hydroxyethyl)pyridiniumbenzolsulfonat, 2-Acetyl-1-(2-hydroxyethyl)pyridiniumbromid, 2-Acetyl-1-(2-hydroxyethyl)-pyridiniumchlorid, 2-Acetyl-1-(2-hydroxyethyl)pyridiniumhydrogensulfat, 2-Acetyl-1-(2-hydroxyethyl)pyridiniumacetat, 2-Acetyl-1-(2-oxopropyl)pyridinium-p-toluolsulfonat, 2-Acetyl-1-(2-oxopropyl)pyridiniumbenzolsulfonat, 2-Acetyl-1-(2-oxopropyl)pyridiniumbromid, 2-Acetyl-1-(2-oxopropyl)pyridiniumchlorid, 2-Acetyl-1-(2-oxopropyl)pyridiniumhydrogensulfat, 2-Acetyl-1-(2-oxopropyl)pyridiniumacetat, 2-Acetyl-1-ethylpyridinium-p-toluolsulfonat, 2-Acetyl-1-ethylpyridiniumbenzolsulfonat, 2-Acetyl-1-ethylpyridiniumbromid, 2-Acetyl-1-ethylpyridiniumchlorid, 2-Acetyl-1-ethylpyridiniumhydrogensulfat, 2-Acetyl-1-ethylpyridiniumacetat, 2-Acetyl-1-(2-methyl-prop-2-enyl)pyridinium-p-toluolsulfonat, 2-Acetyl-1-(2-methyl-prop-2-enyl)pyridiniumbenzolsulfonat, 2-Acetyl-1-(2-methyl-prop-2-enyl)pyridiniumbromid, 2-Acetyl-1-(2-methyl-prop-2-enyl)pyridiniumchlorid, 2-Acetyl-1-(2-methyl-prop-2-enyl)pyridiniumhydrogensulfat, 2-Acetyl-1-(2-methyl-prop-2-enyl)pyridiniumacetat, 2-Acetyl-1-benzylpyridinium-p-toluolsulfonat, 2-Acetyl-1-benzylpyridiniumbenzolsulfonat, 2-Acetyl-1-benzylpyridiniumbromid, 2-Acetyl-1-benzylpyridiniumchlorid, 2-Acetyl-1-benzylpyridiniumhydrogensulfat, 2-Acetyl-1-benzylpyridiniumacetat, 2-Acetyl-1-(2-methoxyethyl)pyridinium-p-toluolsulfonat, 2-Acetyl-1-(2-methoxyethyl)pyridiniumbenzolsulfonat, 2-Acetyl-1-(2-methoxyethyl)pyridiniumbromid, 2-Acetyl-1-(2-methoxyethyl)pyridiniumchlorid, 2-Acetyl-1-(2-methoxyethyl)pyridiniumhydrogensulfat, 2-Acetyl-1-(2-methoxyethyl)pyridiniumacetat, 2,4-Diacetyl-1-methylpyridinium-p-toluolsulfonat, 2,4-Diacetyl-1-methylpyridiniumbenzolsulfonat, 2,4-Diacetyl-1-methylpyridiniumbromid, 2,4-Diacetyl-1-methylpyridiniumchlorid, 2,4-Diacetyl-1-methylpyridiniumhydrogensulfat, 2,4-Diacetyl-1-methylpyridiniumacetat, 2,6-Diacetyl-1-methylpyridinium-p-toluolsulfonat, 2,6-Diacetyl-1-methylpyridiniumbenzolsulfonat, 2,6-Diacetyl-1-methylpyridiniumbromid, 2,6-Diacetyl-1-methylpyridiniumchlorid, 2,6-Diacetyl-1-methylpyridiniumhydrogensulfat, 2,6-Diacetyl-1-methylpyridiniumacetat, 2,6-Diacetyl-1-(2-hydroxyethyl)pyridinium-p-toluolsulfonat, 2,6-Diacetyl-1-(2-hydroxyethyl)pyridiniumbenzolsulfonat, 2,6-Diacetyl-1-(2-hydroxyethyl)pyridiniumbromid, 2,6-Diacetyl-1-(2-hydroxyethyl)pyridiniumchlorid, 2,6-Diacetyl-1-(2-hydroxyethyl)-pyridiniumhydrogensulfat und 2,6-Diacetyl-1-(2-hydroxyethyl)pyridiniumacetat.

Aus dieser Gruppe sind die folgenden Acetylpyridiniumsalze explizit ganz besonders bevorzugt: 2-Acetyl-1-methylpyridinium-p-toluolsulfonat, 2-Acetyl-1-methylpyridiniumbenzolsulfonat, 2-Acetyl-1-methylpyridiniumbromid, 2-Acetyl-1-methylpyridiniumchlorid, 2-Acetyl-1-methylpyridiniumhydrogensulfat, 2-Acetyl-1-methylpyridiniumacetat, 2,6-Diacetyl-1-methylpyridinium-p-toluolsulfonat, 2,6-Diacetyl-1-methylpyridiniumbenzolsulfonat, 2,6-Diacetyl-1-methylpyridiniumbromid, 2,6-Diacetyl-1-methylpyridiniumchlorid, 2,6-Diacetyl-1-methylpyridiniumhydrogensulfat und 2,6-Diacetyl-1-methylpyridiniumacetat.

Soweit nicht explizit anders angegeben, beziehen sich alle nachfolgend genannten Mengenangaben jeweils auf das Gesamtgewicht des anwendungsbereiten Mittels.

Bevorzugt enthalten die erfindungsgemäßen Mittel die 2-Acylpyridinium-Derivate der allgemeinen Struktur (I) bevorzugt in einer Menge von 0,01 bis 25 Gew.-%, insbesondere von 0,1 bis 10 Gew.-%, jeweils bezogen auf das Gesamtgewicht des anwendungsbereiten Mittels.

Als zweiter wesentlicher Inhaltsstoff des erfindungsgemäßen Mittels ist mindestens ein anionisches Tensid, ausgewählt aus Alkylethersulfaten gemäß der Formel R"-(OCH₂CH₂)ₙ-OSO₃M, enthalten, worin R" für eine lineare oder verzweigte, gesättigte oder gegebenenfalls ungesättigte C₈-C₃₀-Alkylkette, n für eine Zahl größer als 2 und M für Wasserstoff oder ein physiologisch verträgliches Kation steht.

Physiologisch verträgliche Kationen im Sinne der Erfindung sind physiologisch verträgliche anorganische Kationen, wobei die Ladung bei mehrwertigen Kationen entsprechend der Stöchiometrie angepasst ist. Bevorzugte anorganische Kationen sind Ammonium, Alkalimetall-Kationen, insbesondere Na⁺ oder K⁺, Erdalkalimetall-Kationen, insbesondere Mg²⁺ oder ½ Ca²⁺, oder Zn²⁺, besonders bevorzugt ist Na⁺.

Physiologisch verträgliche Kationen im Sinne der Erfindung sind weiterhin organische Kationen, wie beispielsweise primäre, sekundäre, tertiäre oder quaternäre Ammonium-Ionen, insbesondere Mono-, Di-, Tri- oder Tetraalkylammonium. Geeignete organische Kationen sind weiterhin Hydroxyalkylammonium-Ionen, wie das Mono- oder das Tri-(2-hydroxyethyl)ammonium-lon (Kationen von Monoethanolamin bzw. Triethanolamin).

Üblicherweise werden Alkylethersulfate ("Ethersulfate") großtechnisch durch SO₃- oder Chlorsulfonsäure (CSA)-Sulfatierung von Fettalkohol- oder Oxoalkoholpolyglycolethern und nachfolgende Neutralisation hergestellt. Erfindungsgemäß bevorzugte Beispiele sind die Sulfate in Form ihrer Natrium- und/oder Magnesiumsalze von ethoxylierten Anlagerungsprodukten von mindestens 2 Ethylenoxid (ausgedrückt durch die Zahl n) an Capronalkohol, Caprylalkohol, 2-Ethylhexylalkohol, Caprinalkohol, Laurylalkohol, Isotridecylalkohol, Myristylalkohol, Cetylalkohol, Stearylalkohol, Isostearylalkohol, Eicosylalkohol oder deren technische Mischungen. Diese fallen beispielsweise bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen an. Bevorzugt sind technische Fettalkohole mit 12 bis 18 Kohlenstoffatomen, wie beispielsweise Kokos-, Palm-, Palmkern- oder Talgfettalkohol.

Bevorzugte Alkylethersulfate der Formel R"-(OCH₂CH₂)ₙ-OSO₃M sind dadurch gekennzeichnet, dass R" für eine gegebenenfalls verzweigte C₈-C₂₂-Alkylgruppe steht, insbesondere für Lauryl-, Myristyl-, Cetyl-, Stearyl- oder einem technischen Gemisch umfassend solche Alkylreste, wie bei Kokos-, Palm-, Palmkern- oder Talgfettalkohol.

Es hat sich herausgestellt, dass sich Alkylethersulfate mit einem hohen Ethoxylierungsgrad besonders gut dazu eignen, eine zusätzliche Verstärkung der Aufhellleistung zu erzielen. Besonders vorteilhaft einsetzbare Akylethersulfate der Formel R"-(OCH₂CH₂)ₙ-OSO₃M zeichnen sich daher dadurch aus, dass n für eine Zahl von 16 bis 50, insbesondere von 20 bis 40 und ganz besonders bevorzugt von 25 bis 35, steht. Die Ethersulfate können dabei sowohl eine kon-ventionelle als auch eine eingeengte Homologenverteilung aufweisen.

Ganz besonders bevorzugt sind Alkylethersulfate der Formel R"-(OCH₂CH₂)ₙ-OSO₃M, worin R" für eine gegebenenfalls verzweigte C₈-C₂₂-Alkylgruppe und n für eine Zahl von 16 bis 50, insbesondere von 20 bis 40 und ganz besonders bevorzugt von 25 bis 35, steht.

Erfindungsgemäß bevorzugte Akylethersulfate sind weiterhin solche Verbindungen gemäß der Formel R"-(OCH₂CH₂)ₙ-OSO₃M, worin R" für eine C₁₀-C₁₈-Alkylgruppe und n für eine Zahl von 25 bis 35 steht.

Besonders bevorzugt ist der Einsatz von Ethersulfaten auf Basis von Addukten von durchschnittlich 30 Mol Ethylenoxid an technische C₁₂-C₁₄- bzw. C₁₂-C₁₈-Kokosfettalkohofraktionen, insbesondere in Form ihrer Natrium- und/oder Magnesiumsalze.

Ein erfindungsgemäß besonders bevorzugtes Alkylethersulfat ist unter der INCI-Bezeichnung Sodium Coceth-30 sulfate geführt und wird unter dem Handelsnamen Disponil^{®} FES 77 als 31-33 Gew.-%ige, wässrige Lösung von der Firma Cognis vertrieben.

Vorzugsweise werden die erfindungsgemäßen Alkylethersulfate innerhalb bestimmter Mengenbereiche eingesetzt. Es sind erfindungsgemäße Mittel bevorzugt, die 0,01 bis 15 Gew.-%, insbesondere 0,1 bis 10 Gew.-% und besonders bevorzugt 0,5 bis 5,0 Gew.-%, jeweils bezogen auf das Gesamtgewicht des anwendungsbereiten Mittels, mindestens eines Alkylethersulfats enthalten.

Als dritter wesentlicher Inhaltsstoff ist Wasserstoffperoxid in dem erfindungsgemäßen Mittel enthalten. Bevorzugt wird Wasserstoffperoxid selbst als wässrige Lösung verwendet. Wasserstoffperoxid kann aber auch in Form einer festen Anlagerungsverbindung von Wasserstoffperoxid an anorganische oder organische Verbindungen, wie beispielsweise Natriumperborat, Natriumpercarbonat, Magnesiumpercarbonat, Natriumpercarbamid, Polyvinylpyrrolidinon n H₂O₂ (n ist eine positive ganze Zahl größer 0), Harnstoffperoxid und Melaminperoxid, eingesetzt werden. Im letztgenannten Fall setzen die Anlagerungsverbindungen in der erfindungsgemäßen Anwendungsmischung Wasserstoffperoxid frei, d. h. diese Mittel enthalten neben der Anlagerungsverbindung im kosmetischen Träger freies Wasserstoffperoxid. Erfindungsgemäß ganz besonders bevorzugt wird das Wasserstoffperoxid dem erfindungsgemäßen Mittel als wässrige Wasserstoffperoxid-Lösung zudosiert. Die Konzentration einer Wasserstoffperoxid-Lösung wird einerseits von den gesetzlichen Vorgaben und andererseits von dem gewünschten Effekt bestimmt; vorzugsweise werden 6 bis 12 gew.-%ige Lösungen in Wasser verwendet. Erfindungsgemäß bevorzugte Mittel sind dadurch gekennzeichnet, dass sie, bezogen auf ihr Gesamtgewicht, 0,01 bis 12 Gew.-%, vorzugsweise 0,1 bis 10 Gew.%, besonders bevorzugt 1 bis 6 Gew.-% Wasserstoffperoxid (berechnet als 100%iges H₂O₂) enthalten.

Unter Berücksichtigung der bisher genannten bevorzugten Ausführungsformen stellt es eine ganz spezielle und ausdrücklich bevorzugte Ausführungsform dar, wenn das Mittel zum Aufhellen von keratinischen Fasern in einem kosmetischen Träger als erste Komponente mindestens eine Verbindung ausgewählt aus der Gruppe, die gebildet wird aus 2-Acetyl-1-methylpyridinium-p-toluolsulfonat, 2-Acetyl-1-methylpyridiniumbenzolsulfonat, 2-Acetyl-1-methylpyridiniumbromid, 2-Acetyl-1-methylpyridiniumchlorid, 2-Acetyl-1-methylpyridiniumhydrogensulfat, 2-Acetyl-1-methylpyridiniumacetat, 2-Acetyl-1-allylpyridinium-p-toluolsulfonat, 2-Acetyl-1-allylpyridinium-benzolsulfonat, 2-Acetyl-1-allylpyridiniumbromid, 2-Acetyl-1-allylpyridiniumchlorid, 2-Acetyl-1-allylpyridiniumhydrogensulfat, 2-Acetyl-1-allylpyridiniumacetat, 2,6-Diacetyl-1-methylpyridinium-p-toluolsulfonat, 2,6-Diacetyl-1-methylpyridiniumbenzolsulfonat, 2,6-Diacetyl-1-methylpyridiniumbromid, 2,6-Diacetyl-1-methylpyridiniumchlorid, 2,6-Diacetyl-1-methylpyridiniumhydrogensulfat und 2,6-Diacetyl-1-methylpyridiniumacetat enthält, als zweite Komponente ein Alkylethersulfat, insbesondere Sodium Coceth-30 sulfate, und als dritte Komponente Wasserstoffperoxid enthält.

Eine ganz besonders bevorzugtes Aufhellmittel ist dadurch gekennzeichnet, dass es eine Kombination aus
0,1 bis 4,0 Gew.-% 2-Acetyl-1-methylpyridinium-p-toluolsulfonat,
0,1 bis 3,0 Gew.-% Sodium Coceth-30 sulfate (Aktivsubstanz) und
0,1 bis 12,0 Gew.-% Wasserstoffperoxid enthält.

Es hat sich im Laufe der Untersuchungen der Anmelderin herausgestellt, dass sich die Blondierleistung der Mittel weiterhin steigern lässt, wenn die Aufhellmittel mindestens ein aromatisches, organisches Lösungsmittel enthalten. Aromatische Lösungsmittel im Sinne der Erfindung sind dabei solche Verbindungen, die eine aromatische Struktureinheit, wie beispielsweise eine Phenylgruppe, in ihrer Strukturformel aufweisen und weiterhin unter Normalbedingungen, also Raumtemperatur und Normaldruck, flüssig sind. Vorzugsweise handelt es sich dabei um carbocyclische Lösungsmittel, die bevorzugt zusätzlich eine Hydroxygruppe tragen. Bevorzugte Beispiele für solche aromatischen Lösungsmittel sind Alkohole, wie Benzylalkohol, 2-Phenylethylalkohol, 1-Phenylethylalkohol, 2-Phenoxyethanol, 3-Methylbenzylalkohol, 2-Methoxybenzylalkohol und 3-Methoxybenzylalkohol. Ein erfindungsgemäß ganz besonders bevorzugtes aromatisches Lösungsmittel ist Benzylalkohol. Es sind dabei erfindungsgemäße Mittel bevorzugt, die 0,01 bis 15 Gew.-%, insbesondere 0,1 bis 10 Gew.-% und besonders bevorzugt 0,5 bis 5,0 Gew.-%, jeweils bezogen auf das Gesamtgewicht des anwendungsbereiten Mittels, mindestens eines aromatischen, organischen Lösungsmittels enthalten.

Untersuchungen zu dieser Anmeldung haben gezeigt, dass der Zusatz einer Aminosäure und/oder eines Oligopeptids die Aufhellleistung des Blondiermittels weiter zu steigern vermag. Eine weitere, bevorzugte Ausführungsform der vorliegenden Erfindung ist daher Mittel, welches dadurch gekennzeichnet ist, dass es zusätzlich mindestens eine Aminosäure und/oder ein Oligopeptid enthält.

Erfindungsgemäß bevorzugte Aminosäuren und Oligopeptide sind aliphatische Aminosäuren sowie Oligopeptide, deren durchschnittlicher, isoelektrischer Punkt (pl) bei etwa 6 liegt. Der isoelektrische Punkt ist als derjenige pH-Wert einer wässrigen Lösung definiert, bei dem sich positive und negative Ladungen einer Substanz mit unterschiedlich geladenen Gruppen (Zwitterionen) gegenseitig ausgleichen. Beispiele für aliphatische Aminosäuren sind Glycin (pl: 5,97), Alanin (pl: 6,02), Valin (pl: 5,96), Leucin (pl: 5,98) oder Isoleucin (pl: 5,94).

Die Aminosäuren und Oligopeptide können den erfindungsgemäßen Mitteln bevorzugt in freier Form zugegeben werden. In einer Reihe von Fällen ist es jedoch auch vorteilhaft, insbesondere die Aminosäuren in Salzform einzusetzen. Ganz besonders bevorzugt ist im erfindungsgemäßen Mittel als Aminosäure Glycin und/oder dessen physiologisch verträgliches Salz enthalten.

Erfindungsgemäß bevorzugte Mittel sind dadurch gekennzeichnet, dass Aminosäuren und Oligopeptide in einer Gesamtmenge die 0,01 bis 15 Gew.-%, insbesondere 0,1 bis 10 Gew.-% und besonders bevorzugt 0,5 bis 5,0 Gew.-%, jeweils bezogen auf das Gesamtgewicht des anwendungsbereiten Mittels, enthalten sind.

Unter Berücksichtigung der bisher genannten bevorzugten Ausführungsformen stellt es eine ganz spezielle und ausdrücklich bevorzugte Ausführungsform dar, wenn das Mittel zum Aufhellen von keratinischen Fasern in einem kosmetischen Träger als erste Komponente mindestens eine Verbindung ausgewählt aus der Gruppe, die gebildet wird aus 2-Acetyl-1-methylpyridinium-p-toluolsulfonat, 2-Acetyl-1-methylpyridiniumbenzolsulfonat, 2-Acetyl-1-methylpyridiniumbromid, 2-Acetyl-1-methylpyridiniumchlorid, 2-Acetyl-1-methylpyridiniumhydrogensulfat, 2-Acetyl-1-methylpyridiniumacetat, 2-Acetyl-1-allylpyridinium-p-toluolsulfonat, 2-Acetyl-1-allylpyridinium-benzolsulfonat, 2-Acetyl-1-allylpyridiniumbromid, 2-Acetyl-1-allylpyridiniumchlorid, 2-Acetyl-1-allylpyridiniumhydrogensulfat, 2-Acetyl-1-allylpyridiniumacetat, 2,6-Diacetyl-1-methylpyridinium-p-toluolsulfonat, 2,6-Diacetyl-1-methylpyridiniumbenzolsulfonat, 2,6-Diacetyl-1-methylpyridiniumbromid, 2,6-Diacetyl-1-methylpyridiniumchlorid, 2,6-Diacetyl-1-methylpyridiniumhydrogensulfat und 2,6-Diacetyl-1-methylpyridiniumacetat enthält, als zweite Komponente ein Alkylethersulfat, insbesondere Sodium Coceth-30 sulfate, und als dritte Komponente Wasserstoffperoxid sowie zusätzlich mindestens eine Verbindung ausgewählt aus Glycin, und Benzylalkohol enthält.

Besonders bevorzugte Aufhellmittel enthalten dabei eine Kombination aus
0,1 bis 4,0 Gew.-% 2-Acetyl-1-methylpyridinium-p-toluolsulfonat,
0,1 bis 10 Gew.-% mindestens eines Alkylethersulfats, insbesondere Sodium Coceth-30 sulfate,
0,1 bis 12,0 Gew.-% Wasserstoffperoxid und
0,1 bis 3,0 Gew.-% Benzylalkohol.

Weiterhin besonders bevorzugte Aufhellmittel enthalten eine Kombination aus
0,1 bis 4,0 Gew.-% 2-Acetyl-1-methylpyridinium-p-toluolsulfonat,
0,1 bis 10 Gew.-% mindestens eines Alkylethersulfats, insbesondere Sodium Coceth-30 sulfate,
0,1 bis 12,0 Gew.-% Wasserstoffperoxid und
0,1 bis 3,0 Gew.-% Glycin.

Blondierprozesse auf Keratinfasern laufen üblicherweise im alkalischen Milieu ab. Um die Keratinfasern und auch die Haut so weit wie möglich zu schonen, ist die Einstellung eines zu hohen pH-Wertes jedoch nicht wünschenswert. Daher ist es bevorzugt, wenn der pH-Wert des verwendungsbereiten Mittels zwischen 7 und 11, insbesondere zwischen 8 und 10,5, liegt. Bei den pH-Werten im Sinne der vorliegenden Erfindung handelt es sich um pH-Werte, die bei einer Temperatur von 22 °C gemessen wurden.

Die zur Einstellung des bevorzugten pH-Wertes erfindungsgemäß verwendbaren Alkalisierungsmittel können aus der Gruppe, die gebildet wird aus Ammoniak, Alkalimetallhydroxiden, Alkanolaminen, Alkalimetallmetasilikaten, Alkalimetallphosphaten und Alkalimetallhydrogenphosphaten ausgewählt werden. Als Alkalimetallionen dienen bevorzugt Lithium, Natrium, Kalium.

Erfindungsgemäß einsetzbare, organische Alkalisierungsmittel werden bevorzugt ausgewählt aus Alkanolaminen aus primären, sekundären oder tertiären Aminen mit einem C₂-C₆-Alkylgrundkörper, der mindestens eine Hydroxylgruppe trägt. Besonders bevorzugte Alkanolamine werden aus der Gruppe ausgewählt, die gebildet 2-Aminoethan-1-ol (Monoethanolamin), 2-Amino-2-methylpropan-1-ol, 2-Amino-2-methyl-propan-1,3-diol und Triethanolamin. Insbesondere bevorzugte Alkanolamine sind Monoethanolamin und Triethanolamin. Bevorzugt sind die Alkanolamine in einer Menge von 0,05 bis 10 Gew.%, insbesondere von 0,5 bis 5 Gew.-%, jeweils bezogen auf das Gesamtgewicht des anwendungsbereiten Mittels, enthalten.

Es hat sich aber im Rahmen der Untersuchungen zur vorliegenden Erfindung herausgestellt, dass weiterhin erfindungsgemäß bevorzugte Mittel dadurch gekennzeichnet sind, dass sie zusätzlich ein anorganisches Alkalisierungsmittel enthalten. Das erfindungsgemäße, anorganische Alkalisierungsmittel wird bevorzugt ausgewählt aus der Gruppe, die gebildet wird aus Natriumhydroxid, Kaliumhydroxid, Calciumhydroxid, Bariumhydroxid, Natriumphosphat, Kaliumphosphat, Natriumsilicat, Kaliumsilicat, Natriumcarbonat und Kaliumcarbonat, bevorzugt aus Natriumhydroxid und Kaliumhydroxid.

Die als erfindungsgemäßes Alkalisierungsmittel einsetzbaren basischen Aminosäuren werden bevorzugt ausgewählt aus der Gruppe, die gebildet wird aus L-Arginin, D-Arginin, D/L-Arginin, L-Lysin, D-Lysin, D/L-Lysin, besonders bevorzugt L-Arginin, D-Arginin, D/L-Arginin als ein Alkalisierungsmittel im Sinne der Erfindung eingesetzt.

Wie bereits erwähnt, können die erfindungsgemäßen Mittel auch direkt vor der Anwendung aus zwei oder mehreren getrennt verpackten Zubereitungen hergestellt werden. Dies bietet sich insbesondere zur Trennung inkompatibler Inhaltsstoffe an, um eine vorzeitige Reaktion zu vermeiden. Um eine vorzeitige, unerwünschte Reaktion der kationischen 2-Acylpyridinium-Derivate der allgemeinen Formel (I) und des Alkylethersulfats mit dem Oxidationsmittel zu verhindern, werden 2-Acylpyridinium-Derivate der allgemeinen Formel (I) und Alkylethersulfat zweckmäßiger vom Oxidationsmittel getrennt voneinander konfektioniert und erst unmittelbar vor der Anwendung in Kontakt gebracht. Ein üblicher Weg besteht daher darin, ein erstes Mittel, welches mindestens ein kationisches 2-Acylpyridinium-Derivat der allgemeinen Formel (I) und mindestens ein Alkylethersulfat enthält, direkt vor der Anwendung mit einem zweiten Mittel, in welchem das oder die efindungsgemäßen Oxidationsmittel enthalten sind, zu vermischen.

Ein weiterer Gegenstand der vorliegenden Erfindung ist daher ein Mittel zum Aufhellen keratinischer Fasern, insbesondere menschlicher Haare, welches unmittelbar vor dem Aufbringen auf das Haar aus einer fließfähigen Zubereitung (A), die die kationischen 2-Acylpyridinium-Derivate der allgemeinen Formel (I) und ein Alkylethersulfat enthält, und einer Oxidationsmittelzubereitung (B), enthaltend mindestens ein Oxidationsmittel, ausgewählt aus Wasserstoffperoxid und/oder dessen Anlagerungsverbindungen an organische oder anorganische Verbindungen, erhalten wird.

Eine weitere Ausführungsform der vorliegenden Erfindung ist weiterhin ein Mittel zum Aufhellen keratinischer Fasern, insbesondere menschlicher Haare, welches unmittelbar vor dem Aufbringen auf das Haar aus einer fließfähigen Zubereitung (A), die die kationischen 2-Acylpyridinium-Derivate der allgemeinen Formel (I) enthält, und einer Oxidationsmittelzubereitung (B), enthaltend mindestens und ein Alkylethersulfat und ein Oxidationsmittel, ausgewählt aus Wasserstoffperoxid und/oder dessen Anlagerungsverbindungen an organische oder anorganische Verbindungen, erhalten wird.

Die Oxidationsmittelzubereitung (B) ist vorzugsweise eine wässrige, fließfähige Oxidationsmittelzubereitung. Dabei sind bevorzugte erfindungsgemäße Mittel zum Aufhellen keratinischer Fasern dadurch gekennzeichnet, dass die fließfähige Oxidationsmittelzubereitung (B) - bezogen auf ihr Gewicht - 40 bis 90 Gew.-%, vorzugsweise 50 bis 85 Gew.-%, bevorzugt 55 bis 80 Gew.-%, weiter bevorzugt 60 bis 77,5 Gew.-% und insbesondere 65 bis 75 Gew.-% Wasser enthält.

Erfindungsgemäß kann aber das Oxidationsfärbemittel auch zusammen mit einem Katalysator auf das Haar aufgebracht werden. Solche Katalysatoren sind z. B. bestimmte Enzyme, Iodide, Chinone oder Metallionen. Geeignete Enzyme sind z. B. Peroxidasen, die die Wirkung geringer Mengen an Wasserstoffperoxid deutlich verstärken können. Besonders geeignete Katalysatoren für die Oxidation der Farbstoffvorläufer sind die sogenannten 2-Elektronen-Oxidoreduktasen in Kombination mit den dafür spezifischen Substraten, z. B. Pyranose-Oxidase, Glucose-Oxidase, Glycerin-Oxidase, Pyruvat-Oxidase, Alkohol-Oxidase, Lactat-Oxidase, Tyrosinase-Oxidase, Uricase, Cholinoxidase, Aminosäure-Oxidase. Ein Einsatz bestimmter Metallionen oder -komplexe kann ebenfalls bevorzugt sein, um verbesserte Bleichwirkungen zu erzielen. Geeignete Metallionen sind beispielsweise Zn²⁺, Cu²⁺, Fe²⁺, Fe³⁺, Mn²⁺, Mn⁴⁺, Li⁺, Mg²⁺, Ca²⁺, Ce⁴⁺, V³⁺, Co²⁺, Ru³⁺ und Al³⁺, insbesondere Zn²⁺, Cu²⁺ und Mn²⁺. Besonders bevorzugte Mittel enthalten diese Metallionen zu 0,0001 bis 2,5 Gew.-%, vorzugsweise 0,001 bis 1 Gew.-%, bezogen auf das Gesamtgewicht des erfindungsgemäßen Mittels.

Weiterhin hat es sich als vorteilhaft erweisen, wenn die Oxidationsmittelzubereitungen (B) mindestens einen Stabilisator oder Komplexbildner enthalten. Besonders bevorzugte Stabilisatoren sind Phenacetin, Alkalibenzoate (Natriumbenzoat) und Salicylsäure.

Erfindungsgemäß bevorzugt ist auch der Einsatz von sogenannten Komplexbildnern. Komplexbilder sind Stoffe, die Metallionen komplexieren können. Bevorzugte Komplexbildner sind sogenannte Chelatkomplexbildner, also Stoffe, die mit Metallionen cyclische Verbindungen bilden, wobei ein einzelner Ligand mehr als eine Koordinationsstelle an einem Zentralatom besetzt, d. h. mindestens "zweizähnig" ist. Die Zahl der gebundenen Liganden hängt von der Koordinationszahl des zentralen Ions ab. Gebräuchliche und im Rahmen der vorliegenden Efindung bevorzugte Chelatkomplexbildner sind beispielsweise Polyoxycarbonsäuren, Polyamine, Ethylendiamintetraessigsäure (EDTA), Nitrilotriessigsäure (NTA) und Hydroxyethandiphosphonsäuren bzw. deren Alkalisalze. Erfindungsgemäß bevorzugte Komplexbildner sind stickstoffhaltigen Polycarbonsäuren, insbesondere EDTA, und Phosphonate, vorzugsweise Hydroxyalkan- bzw. Aminoalkanphosphonate und insbesondere 1-Hydroxyethan-1,1-diphosphonat (HEDP) bzw. dessen Di-oder Tetranatriumsalz und/oder Ethylendiamintetramethylenphosphonat (EDTMP) bzw. dessen Hexanatriumsalz und/oder Diethylentriaminpentamethylenphosphonat (DTPMP) bzw. dessen Hepta-oder Octanatriumsalz.

Die Oxidationsmittelzubereitung enthält neben dem eigentlichen Oxidationsmittel weitere Hilfs-und Zusatzstoffe. So hat es sich erfindungsgemäß als bevorzugt erwiesen, wenn die Oxidationsmittelzubereitung mindestens ein Verdickungsmittel enthält. Bezüglich dieser Verdickungsmittel bestehen keine prinzipiellen Einschränkungen. Es können sowohl organische als auch rein anorganische Verdickungsmittel zum Einsatz kommen.

Gemäß einer ersten bevorzugten Ausführungsform handelt es sich bei dem Verdickungsmittel um ein anionisches, synthetisches Polymer, wie beispielsweise unter den Warenzeichen Carbopol^{®}, Rheothik^{®}11-80, Aculyn^{®} 22, Aculyn^{®} 33, Structure^{®} 2001, Structure^{®} 3001, Sepigel^{®}305, Simulgel^{®} 600, Stabileze^{®} QM, Latekoll^{®} D im Handel erhältlich.

Gemäß einer weiteren Ausführungsform handelt es sich bei dem Verdickungsmittel um einen kationisches synthetisches Polymer. Ein besonders geeignetes Homopolymer ist das Polyquaternium-37 sowie die unter den Handelsbezeichnungen Salcare^{®} SC 92, Salcare^{®} SC 95, Salcare^{®} SC 96, Jaguar^{®} HP8, Jaguar^{®} HP60, Jaguar^{®} HP120, Jaguar^{®} DC 293 und Jaguar^{®} HP105 vertriebenen Polymere.

Weiterhin geeignete natürliche Verdickungsmittel sind ebenfalls bereits aus dem Stand der Technik bekannt. Gemäß dieser Ausführungsform bevorzugt sind weiterhin Biosaccharidgums mikrobiellen Ursprungs, wie Skleroglucangums oder Xanthangums, Gums aus pflanzlichen Exsudaten, wie Gummi arabicum, Ghatti-Gummi, Karaya-Gummi, Tragant-Gummi, Carrageen-Gummi, Agar-Agar, Johannisbrotkernmehl, Pektine, Alginate, Stärke-Fraktionen und Derivate wie Amylose, Amylopektin und Dextrine, Cellulosederivate, wie Methylcellulose, Carboxyalkylcellulosen und Hydroxyalkylcellulosen (Cellosize^{®}, Natrosol^{®}, Blanose^{®}, Aquasorb^{®}, Ambergum^{®} und Cellgon^{®}).

Aber auch nichtionische, vollsynthetische Polymere, wie Polyvinylalkohol oder Polyvinylpyrrolidinon, sind als erfindungsgemäße Verdickungsmittel einsetzbar.

Als anorganische Verdickungsmittel haben sich Schichtsilikate (polymere, kristalline Natriumdisilicate) als besonders geeignet im Sinne der vorliegenden Erfindung erwiesen. Insbesondere Tone, insbesondere Magnesium Aluminium Silicate, wie beispielsweise Bentonit, besonders Smektite, wie Montmorillonit oder Hectorit, die gegebenenfalls auch geeignet modifiziert sein können, und synthetische Schichtsilikate, wie beispielsweise das von der Firma Süd Chemie unter der Handelsbezeichnung Optigel^{®} vertriebene Magnesiumschichtsilikat, sind bevorzugt.

Zur weiteren Steigerung der Aufhellleistung können der erfindungsgemäßen Zusammensetzung zusätzlich mindestens eine gegebenenfalls hydratisierte SiO₂-Verbindung zugesetzt. Obwohl bereits geringe Mengen der gegebenenfalls hydratisierten SiO₂-Verbindungen die Aufhellleistung erhöhen, kann es erfindungsgemäß bevorzugt sein, die gegebenenfalls hydratisierten SiO₂-Verbindungen in Mengen von 0,05 Gew.-% bis 15 Gew.-%, besonders bevorzugt in Mengen von 0,15 Gew.-% bis 10 Gew.-% und ganz besonders bevorzugt in Mengen von 0,2 Gew.-% bis 5 Gew.-%, jeweils bezogen auf die erfindungsgemäße wasserfreie Zusammensetzung, einzusetzen. Die Mengenangaben geben dabei jeweils den Gehalt der SiO₂-Verbindungen (ohne deren Wasseranteil) in den Mitteln wieder.

Hinsichtlich der gegebenenfalls hydratisierten SiO₂-Verbindunge unterliegt die vorliegende Erfindung prinzipiell keinen Beschränkungen. Bevorzugt sind Kieselsäuren, deren Oligomeren und Polymeren sowie deren Salze. Bevorzugte Salze sind die Alkalisalze, insbesondere die Kalium und Natriumsalze. Die Natriumsalze sind ganz besonders bevorzugt. Die gegebenenfalls hydratisierten SiO₂-Verbindungen können in verschiedenen Formen vorliegen. Erfindungsgemäß bevorzugt werden die SiO₂-Verbindunge in Form von Kieselgelen (Silicagel) oder besonders bevorzugt als Wasserglas eingesetzt. Diese SiO₂-Verbindunge können teilweise in wässriger Lösung vorliegen. Erfindungsgemäß besonders bevorzugte Wassergläser werden unter anderem von der Firma Henkel unter den Bezeichnungen Ferrosil^{®} 119, Natronwasserglas 40/42, Portil^{®} A, Portil^{®} AW und Portil^{®} W und von der Firma Akzo unter der Bezeichnung Britesil^{®} C20 vertrieben.

Vorzugsweise sind die Oxidationsmittelzubereitung (B) und die Zubereitung (A) als fließfähige Zubereitungen konfektioniert.

Vorzugsweise wird den fließfähigen Zubereitungen (A) und/oder (B) weiterhin ein Emulgator bzw. ein Tensid zugesetzt, wobei oberflächenaktive Substanzen je nach Anwendungsgebiet als Tenside oder als Emulgatoren bezeichnet werden und aus anionischen, kationischen, zwitterionischen, amphoteren und nichtionischen Tensiden und Emulgatoren ausgewählt sind. Diese Stoffe werden nachfolgend ausführlich beschrieben.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie beispielsweise eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono, Di- und Trialkanolammoniumsalze, lineare und verzweigte Fettsäuren (Seifen); Ethercarbonsäuren; Acylsarcoside; Acyltauride; Acylisethionate; Sulfobernsteinsäuremono- und -dialkylester und Sulfobernsteinsäuremono-alkylpolyoxyethylester; lineare Alkansulfonate; lineare α-Olefinsulfonate; Sulfonate ungesättigter Fettsäuren; α-Sulfofettsäuremethylester ; Alkylsulfate; Gemische oberflächenaktiver Hydroxysulfonate; sulfatierte Hydroxyalkylpolyethylen- und/oder Hydroxyalkylenpropylenglykolether; Ester der Weinsäure und Zitronensäure mit Anlagerungsprodukten von Ethylenoxid und/oder Propylenoxid an Fettalkohole; Alkyl- und/oder Alkenyletherphosphate; sulfatierte Fettsäurealkylenglykolester; Monoglyceridsulfate und Monoglyceridethersulfate.

Bevorzugte anionische Tenside sind Alkylsulfate und Ethercarbonsäuren mit 10 bis 18 C-Atomen in der Alkylgruppe und bis zu 12 Glykolethergruppen im Molekül.

Als zwitterionische Tenside werden solche oberflächenaktiven Verbindungen bezeichnet, die im Molekül mindestens eine quartäre Ammoniumgruppe und mindestens eine Carboxylat-, Sulfonat-oder Sulfat-Gruppe tragen. Besonders geeignete zwitterionische Tenside sind die sogenannten Betaine wie die N-Alkyl-N,N-dimethylammonium-glycinate, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter amphoteren Tensiden werden solche oberflächenaktiven Verbindungen verstanden, die außer einer C₈-C₂₄-Alkyl- oder C₈-C₂₄-Acylgruppe im Molekül mindestens eine freie Aminogruppe und mindestens eine COOH- oder SO₃H-Gruppe enthalten und zur Ausbildung innerer Salze befähigt sind. Beispiele für geeignete amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte amphotere Tenside sind N-Kokosalkylaminopropionat, Kokosacylaminoethylaminopropionat und Acylsarcosin.

Weiterhin hat es sich als vorteilhaft erwiesen, wenn die erfindungsgemäßen Färbe- und Aufhellmittel weitere, nichtionogene grenzflächenaktive Stoffe, enthalten. Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
Anlagerungsprodukte von Ethylenoxid und/oder an lineare und verzweigte Fettalkohole, Fettsäuren und Alkylphenole mit 8 bis 15 C-Atomen, mit einem Methyl- oder C₂-C₆-Alkylrest endgruppenverschlossene Anlagerungsprodukte an lineare und verzweigte Fettalkohole, Fettsäuren und Alkylphenole; Polyglycerinester (wie Handelsprodukte Lameform^{®}TGI und Dehymuls^{®}PGPH (Henkel)); Polyolfettsäureester (wie Handelsprodukte Hydagen^{®} HSP oder Sovermol-Typen (Cognis)); höher alkoxylierte, bevorzugt propoxylierte und insbesondere ethoxylierte, Mono-, Di-und Triglyceride; Aminoxide; Sorbitanfettsäureester und deren Anlagerungeprodukte von Ethylenoxid (wie Polysorbate); Zuckerfettsäureester und deren Anlagerungsprodukte; Anlagerungsprodukte von Ethylenoxid an Fettsäurealkanolamide und Fettamine; Fettsäure-N-alkylglucamide sowie Alkylpolyglykoside .

Als nichtionische Tenside eignen sich insbesondere C₈-C₂₂-Alkylmono- und oligoglycoside und deren ethoxylierte Analoga. Als weitere bevorzugte nichtionische Tenside haben sich die Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

Die zusätzlichen anionischen, nichtionischen, zwitterionischen oder amphoteren Tenside werden in Mengen von 0,1 bis 45 Gew.%, bevorzugt 1 bis 30 Gew.% und ganz besonders bevorzugt von 1 bis 15 Gew.%, bezogen auf die Gesamtmenge des anwendungsbereiten Mittels, eingesetzt.

Erfindungsgemäß bevorzugt sind ebenfalls kationische Tenside vom Typ quaternärer Ammoniumverbindungen, der Esterquats und der Amidoamine. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf. Weitere erfindungsgemäß verwendbare kationische Tenside stellen die quaternisierten Proteinhydrolysate dar. Die Alkylamidoamine werden üblicherweise durch Amidierung natürlicher oder synthetischer Fettsäuren und Fettsäureschnitte mit Dialkylaminoaminen hergestellt und zeichnen sich neben einer guten konditionierenden Wirkung speziell durch ihre gute biologische Abbaubarkeit aus. Eine erfindungsgemäß besonders geeignete Verbindung aus dieser Substanzgruppe stellt das unter der Bezeichnung Tegoamid^{®} S 18 im Handel erhältliche Stearamidopropyl-dimethylamin dar. Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen (wie Handelsprodukte Stepantex^{®}, Dehyquart^{®} und Armocare^{®}). Die kationischen Tenside sind in den erfindungsgemäß verwendeten Mitteln bevorzugt in Mengen von 0,05 bis 10 Gew.%, bezogen auf das gesamte Mittel, enthalten. Mengen von 0,1 bis 5 Gew.-% sind besonders bevorzugt.

In einer bevorzugten Ausführungsform können nicht-ionische, zwitterionische und/oder amphotere Tenside sowie deren Mischungen bevorzugt sein.

Für die starke Aufhellung sehr dunklen Haares ist der alleinige Einsatz von Wasserstoffperoxid oder dessen Anlagerungsprodukten an organische beziehungsweise anorganische Verbindungen oftmals nicht ausreichend. In diesen Fällen wird in der Regel eine Kombination aus Wasserstoffperoxid und Persulfaten bzw. Peroxodisulfaten eingesetzt. Es hat sich gezeigt, dass die Beimengung der erfindungsgemäßen 2-Acylpyridinium-Derivate der allgemeinen Struktur (I) und von mindestens einem Alkylethersulfat nicht nur bei Wasserstoffperoxid allein, sondern auch bei einer Kombination aus Wasserstoffperoxid und Persulfatsalzen bzw. Peroxodisulfatsalzen in einer Steigerung des Aufhellvermögens resultiert.

Daher kann es, sollte der Verbraucher den Wunsch nach einer sehr starken Blondierung verspüren, in einer weiteren Ausführungsform bevorzugt sein, wenn neben der kationischen 2-Acylpyridiniumverbindung der allgemeinen Struktur (I), einem Alkylethersulfat und Wasserstoffperoxid zusätzlich mindestens ein anorganisches Persulfatsalz bzw. Peroxodisulfatsalz in dem Mittel zum Aufhellen der keratinischen Fasern enthalten ist. Bevorzugte Peroxodisulfatsalze sind Ammoniumperoxodisulfat, Kaliumperoxodisulfat und Natriumperoxodisulfat. Die Peroxodisulfatsalze können in einer Menge von 0,1 bis 25 Gew.-%, insbesondere in einer Menge von 0,5 bis 15 Gew.-%, bezogen auf das Gesamtgewicht des anwendungsbereiten Mittels, enthalten sein.

Der Einsatz von Persulfatsalzen bzw. Peroxodisulfatsalzen erfolgt in der Regel in Form eines gegebenenfalls entstaubten Pulvers oder eines in Form gepressten Formkörpers. Um einen vorzeitigen Abbau der erfindungsgemäßen 2-Acylpyridinium-Derivate durch den Kontakt mit den Persulfaten bzw. Peroxodisulfaten zu vermeiden, ist es erfindungsgemäß bevorzugt, die Persulfate bzw. Peroxodisulfate als separat verpackte Komponente (C) zur Verfügung zu stellen.

In diesem Zusammenhang ist ein aus 3 Komponenten bestehendes Mittel zum Aufhellen von menschlichen Haaren ein weiterer Gegenstand der vorliegenden Erfindung. Dieses Mittel wird unmittelbar vor dem Aufbringen auf das Haar durch sorgfältiges Vermischen einer fließfähigen Zubereitung (A), die die kationischen 2-Acylpyridinium-Derivate der allgemeinen Formel (I) und mindestens ein Alkylethersulfat enthält, einer Oxidationsmittelzubereitung (B), enthaltend mindestens ein Oxidationsmittel, ausgewählt aus Wasserstoffperoxid und/oder dessen Anlagerungsverbindungen an organische oder anorganische Verbindungen, und zusätzlich einer dritten, in Pulverform vorliegenden Zubereitung (C), welche mindestens ein anorganisches Persulfatsalz bzw. Peroxodisulfatsalz enthält, hergestellt.

Ebenso ist es möglich, das Alkylethersulfat, gemeinsam mit dem Oxidationsmittel zu konfektionieren. Eine weitere Ausführungsform der vorliegenden Erfindung ist daher ein Mittel, welches unmittelbar vor dem Aufbringen auf das Haar durch sorgfältiges Vermischen einer fließfähigen Zubereitung (A), die die kationischen 2-Acylpyridinium-Derivate der allgemeinen Formel (I) enthält, einer Oxidationsmittelzubereitung (B), enthaltend mindestens ein Alkylethersulfat und mindestens ein Oxidationsmittel, ausgewählt aus Wasserstoffperoxid und/oder dessen Anlagerungsverbindungen an organische oder anorganische Verbindungen, und zusätzlich einer dritten, in Pulverform vorliegenden Zubereitung (C), welche mindestens ein anorganisches Persulfatsalz bzw. Peroxodisulfatsalz enthält, hergestellt wird.

Die Mischung der Zubereitungen (A) und (B) bzw. gegebenenfalls der Zubereitungen (A), (B) und (C) vor der Anwendung führt zu einer Anwendungsmischung, die ein erfindungsgemäßes Mittel mit den drei zwingenden Inhaltsstoffen ist.

Als zusätzliche Bleichkraftverstärker können im Rahmen dieser Erfindung Verbindungen, die unter Perhydrolysebedingungen aliphatische Peroxocarbonsäuren und/oder substituierte Perbenzoesäure ergeben, Kohlensäurederivate, Alkylcarbonate und -carbamate sowie Silylcarbonate und -carbamate eingesetzt werden. Als weitere zusätzliche Bleichverstärker können in den erfindungsgemäßen Zusammensetzungen bevorzugt mindestens eine Verbindung ausgewählt aus Essigsäure, Milchsäure, Weinsäure, Zitronensäure, Salicylsäure und ortho-Phthalsäure, enthalten sein. Die neben oder anstelle von Peroxoverbindungen eingesetzten Bleichkraftverstärker sind in den erfindungsgemäßen, kosmetischen Mitteln bevorzugt in Mengen von 0,05 bis 10 Gew.-%, insbesondere in Mengen von 0,2 bis 5 Gew.-%, jeweils bezogen auf das Gesamtgewicht des anwendungsbereiten Mittels, enthalten.

Obwohl prinzipiell keine Einschränkungen hinsichtlich der Formulierung der Blondierzubereitung (C) bestehen, hat es sich erfindungsgemäß als bevorzugt erwiesen, wenn die Zubereitung (C) wasserfrei formuliert ist. Wasserfrei im Sinne der vorliegenden Erfindung bedeutet einen Wassergehalt bezogen auf die Zubereitung (C) von weniger als 5 Gew.-%, insbesondere von weniger als 2 Gew.-%. Blondierzubereitungen, die weniger als 0,1 Gew.-% Wasser, enthalten können erfindungsgemäß besonders bevorzugt sein. Die Zubereitung (C) ist vorzugsweise als Pulver oder als wasserfreie Paste formuliert. Im Falle der Formulierung als wasserfreie Paste hat es sich als besonders bevorzugt erwiesen, wenn die Zubereitung (C) mindestens einen nicht hydroxylierten Fettsäureester mit einem Schmelzpunkt von höchstens 50°C, insbesondere von höchstens 30°C, und/oder mindestens eine C₁₀-C₃₀-Fettsäure mit mindestens einer zusätzlichen Hydroxygruppe und/oder ein Derivat davon, enthält.

Die erfindungsgemäßen Mittel können nicht nur als reine Aufhellmittel, d.h. als sogenannte Blondiermittel, sondern auch als Färbe- und Aufhellmittel bereitgestellt werden, die gleichzeitig mit der Aufhellung auch eine Färbung der Keratinfasern bewirken.

Die erfindungsgemäßen Mittel können daher zusätzlich auch Farbstoffe und/oder Farbstoffvorprodukte enthalten und somit als Mittel bereitgestellt werden, die gleichzeitig aufhellend und färbend wirken. Solche Mittel werden nachfolgend als "Färbemittel", als "aufhellende Färbemittel" oder als "Färbe- und Aufhellmittel" bezeichnet. Zur Bereitstellung farbverändernder kosmetischer Mittel, insbesondere für die Haut oder keratinhaltige Fasern wie beispielsweise menschliche Haare, kennt der Fachmann je nach Anforderungen an die Färbung diverse Färbesysteme.

In einer Ausführungsform zur Farbveränderung ist der Gegenstand der vorliegenden Erfindung mit mindestens einer farbverändernden Komponente kombinierbar. Die farbverändernden Komponenten im Sinne der vorliegenden Erfindung werden bevorzugt ausgewählt (1) aus mindestens einem Oxidationsfarbstoffvorprodukt und/oder (2) aus mindestens einem direktziehenden Farbstoff und/oder (3) aus mindestens einer Vorstufe naturanaloger Farbstoffe.

Erfindungsgemäß bevorzugte Mittel zum Färben und/oder Aufhellen keratinischer Fasern sind demnach dadurch gekennzeichnet, dass sie mindestens ein Oxidationsfarbstoffvorprodukt vom Entwicklertyp und/oder Kupplertyp enthalten.

Bevorzugte Entwicklerkomponenten werden ausgewählt aus mindestens einer Verbindung aus der Gruppe, die gebildet wird aus p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)-propyl]amin, N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(1,2-dihydroxyethyl)-phenol und 4-Amino-2-(diethylaminomethyl)phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin, sowie den physiologisch verträglichen Salzen dieser Verbindungen. Besonders bevorzugte Entwicklerkomponenten sind dabei p-Toluylendiamin, 2-(2-Hydroxyethyl)-p-phenylendiamin, 2-Methoxymethyl-p-phenylendiamin, N-(4-Amino-3-methylphenyl)-N-[3-(1H-imidazol-1-yl)propyl]-amin, und/oder 4,5-Diamino-1-(2-hydroxyethyl)-pyrazol sowie deren physiologisch verträglichen Salze. Die Entwicklerkomponenten werden bevorzugt in einer Menge von 0,005 bis 20 Gew.-%, vorzugsweise 0,1 bis 5 Gew.-%, jeweils bezogen auf das anwendungsbereite Oxidationsfärbemittel, verwendet.

Kupplerkomponenten bilden im Rahmen der oxidativen Färbung allein keine signifikante Färbung aus, sondern benötigen stets die Gegenwart von Entwicklerkomponenten. Daher ist es erfindungsgemäß bevorzugt, dass bei Verwendung mindestens einer Kupplerkomponente zusätzlich mindestens eine Entwicklerkomponente zum Einsatz kommt.

Erfindungsgemäß bevorzugte Kupplerkomponenten werden ausgewählt unter 3-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)ethanol, 1,3-Bis-(2,4-diaminophenoxy)propan, 1-Methoxy-2-amino-4-(2-hydroxyethylamino)benzol, 1,3-Bis-(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2-hydroxyethyl)aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder Gemischen dieser Verbindungen oder den physiologisch verträglichen Salzen der vorgenannten Verbindungen. Besonders bevorzugt ist Resorcin, 2-Methylresorcin, 5-Amino-2-methylphenol, 3-Aminophenol, 2-(2,4-Diaminophenoxy)ethanol, 1,3-Bis(2,4-diaminophenoxy)propan, 1-Methoxy-2-amino-4-(2'-hydroxyethylamino)benzol, 2-Amino-3-hydroxypyridin und 1-Naphthol sowie eines deren physiologisch verträglichen Salze. Die Kupplerkomponenten werden bevorzugt in einer Menge von 0,005 bis 20 Gew.-%, vorzugsweise 0,1 bis 5 Gew.-%, jeweils bezogen auf das anwendungsbereite Oxidationsfärbemittel, verwendet.

Weiterhin können die erfindungsgemäßen Mittel mindestens einen direktziehenden Farbstoff enthalten. Dabei handelt sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone oder Indophenole. Die direktziehenden Farbstoffe werden jeweils bevorzugt in einer Menge von 0,001 bis 20 Gew.-%, bezogen auf die gesamte Anwendungszubereitung, eingesetzt. Die Gesamtmenge an direktziehenden Farbstoffen beträgt vorzugsweise höchstens 20 Gew.-%. Direktziehende Farbstoffe können in anionische, kationische und nichtionische direktziehende Farbstoffe unterteilt werden.

Als anionische direktziehende Farbstoffe eignen sich insbesondere FD&C Yellow No. 6, Acid Yellow 1, Acid Yellow 3, Acid Yellow 9, Acid Yellow 23, Acid Yellow 36, Acid Yellow 73, Acid Orange 3, Acid Orange 6, Acid Orange 7, Acid Orange 24, Acid Red 4, Acid Red 14, Acid Red 18, Acid Red 27, Acid Red 33, Acid Red 35, Acid Red 51, Acid Red 52, Acid Red 73, Acid Red 87, Acid Red 92, Acid Red 95, Acid Red 184, Acid Red 195, Pigment Red 57:1, FD&C Red No. 4, Acid Green 25, Acid Green 50, Acid Blue 1, Acid Blue 3, Acid Blue 7, Acid Blue 9, Acid Blue 25, Acid Blue 62, Acid Blue 74, Acid Violet 9, Acid Violet 43, Acid Brown 13, Acid Black 1, Acid Black 52), Food Black No. 1, 3',3",5',5"-Tetrabromphenolsulfonphthalein (Bromphenolblau). Bevorzugte anionische direktziehende Farbstoffe sind die unter den internationalen Bezeichnungen bzw. Handelsnamen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1 und Acid Black 52 bekannten Verbindungen.

Als kationische direktziehende Farbstoffe eignen sich Basic Blue 6, Basic Blue 7, Basic Blue 8, Basic Blue 9, Basic Blue 26, Basic Blue 41, Basic Blue No. 99, Basic Violet 1, Basic Violet 2, Basic Violet 3, Basic Violet 10, Basic Violet 14, Basic Brown 4, Basic Brown 16, 1-[(4-Amino-2-nitrophenyl)azo]-7-(trimethylammonio)-2-naphtholchlorid, Basic Brown 17, Basic Orange 69, Basic Red 2, Basic Red 22, Basic Red 76, Basic Yellow 2, Basic Yellow 11, Basic Yellow 57, Basic Green 1, Basic Green 4, 1-(2-Morpholiniumpropylamino)-4-hydroxy-9,10-anthrachinonmethylsulfat, 1-[(3-(Dimethyl-propylaminium)-propyl)amino]-4-(methylamino)-9,10-anthrachinonchlorid und direktziehende Farbstoffe, die einen Heterocyclus enthalten, der mindestens ein quaternäres Stickstoffatom aufweist. Bevorzugte kationische direktziehende Farbstoffe sind dabei kationische Triphenylmethanfarbstoffe, wie beispielsweise Basic Blue 7, Basic Blue 26, Basic Violet 2 und Basic Violet 14, aromatischen Systeme, die mit einer quaternären Stickstoffgruppe substituiert sind, wie Basic Yellow 57, Basic Red 76, Basic Blue 99, Basic Brown 16 und Basic Brown 17, sowie direktziehende Farbstoffe, die einen Heterocyclus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden. Die Verbindungen, die auch unter den Bezeichnungen Basic Yellow 87, Basic Orange 31 und Basic Red 51 bekannt sind, sind ganz besonders bevorzugte kationische direktziehende Farbstoffe. Die kationischen direktziehenden Farbstoffe, die unter dem Warenzeichen Arianor^{®} vertrieben werden, sind erfindungsgemäß besonders bevorzugte kationische direktziehende Farbstoffe.

Als nichtionische direktziehende Farbstoffe eignen sich insbesondere nichtionische Nitro- und Chinonfarbstoffe und neutrale Azofarbstoffe. Bevorzugte nichtionische direktziehende Farbstoffe sind die unter den internationalen Bezeichnungen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)-aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)-amino-5-chlor-2-nitrobenzol, 4-Amino-3-nitrophenol, 1-(2'-Ureidoethyl)amino-4-nitrobenzol, 2-[(4-Amino-2-nitrophenyl)amino]-benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Es ist nicht erforderlich, dass die direktziehenden Farbstoffe jeweils einheitliche Verbindungen darstellen. Vielmehr können, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen. Weiterhin können als direktziehende Farbstoffe auch in der Natur vorkommende Farbstoffe eingesetzt werden, wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu und Alkannawurzel enthalten sind.

Als Farbstoffvorstufen naturanaloger Farbstoffe werden bevorzugt solche Indole und Indoline eingesetzt, die mindestens zwei Gruppen ausgewählt aus Hydroxy- und/oder oder Aminogruppen, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. In einer weiteren Ausführungsform enthalten die Färbemittel mindestens ein Indol- und/oder Indolinderivat. Erfindungsgemäße Zusammensetzungen, die Vorstufen naturanaloger Farbstoffe enthalten, werden bevorzugt als luftoxidative Färbemittel verwendet. In dieser Ausführungsform werden die besagten Zusammensetzungen folglich nicht mit einem zusätzlichen Oxidationsmittel versetzt. Die Farbstoffvorstufen naturanaloger Farbstoffe werden jeweils bevorzugt in einer Menge von 0,001 bis 5 Gew.-%, bezogen auf die gesamte Anwendungszubereitung, eingesetzt. Die Gesamtmenge an direktziehenden Farbstoffen beträgt vorzugsweise höchstens 3 Gew.-%.Besonders bevorzugte Derivate des Indolins sind 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin sowie 5,6-Dihydroxyindolin-2-carbonsäure, insbesondere das 5,6-Dihydroxyindolin. Besonders bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, insbesondere 5,6-Dihydroxyindol.

Ferner können die efindungsgemäßen Mittel weitere Wirk-, Hilfs- und Zusatzstoffe enthalten, wie beispielsweise
nichtionische Polymere (wie Vinylpyrrolidinon/Vinylacrylat-Copolymere, Polyvinylpyrrolidinon, Vinylpyrrolidinon/Vinylacetat-Copolymere, Polyethylenglykole und Polysiloxane); Silikone wie flüchtige oder nicht flüchtige, geradkettige, verzweigte oder cyclische, vernetzte oder nicht vernetzte Polyalkylsiloxane (wie Dimethicone oder Cyclomethicone), Polyarylsiloxane und/oder Polyalkylarylsiloxane, insbesondere Polysiloxane mit organofunktionelle Gruppen, wie Amodimethicone, Dimethiconcopolyole, lineare Polysiloxan(A)-Polyoxyalkylen(B)-Blockcopolymere, gepfropften Silikonpolymere (wie Abil B 8832 der Firma Degussa); kationische Polymere (wie quaternisierte Celluloseether, Polysiloxane mit quaternären Gruppen, Dimethyldiallylammoniumchlorid-Polymere, Acrylamid-Dimethyldiallyl-ammoniumchlorid-Copolymere, mit Diethylsulfat quaternierte Dimethylamino-ethylmethacrylat-Vinylpyrrolidinon-Copolymere, Vinylpyrrolidinon-Imidazolinium-methochlorid-Copolymere und quaternierter Polyvinylalkohol); zwitterionische und amphotere Polymere (wie Acrylamidopropyl-trimethylammoniumchlorid/Acrylat-Copolymere und Octylacrylamid/Methyl-methacrylat/tert-Butylaminoethylmethacrylat/2-Hydroxypropylmethacrylat-Copolymere, Diallyldimethylammoniumchlorid/Acrylat-Copolymere, t-Butylaminoethylmethacrylat/N-(1,1,3,3-Tetramethylbutyl)acrylamid/Acrylat(/Methacrylat)-Copolymere) ; anionische Polymere (wie Polyacrylsäuren, vernetzte Polyacrylsäuren, Vinylacetat/Crotonsäure-Copolymere, Vinylpyrrolidinon/Vinylacrylat-Copolymere, Vinylacetat/Butylmaleat/lsobornylacrylat-Copolymere, Methylvinylether/Malein-säureanhydrid-Copolymere und Acrylsäure/Ethylacrylat/N-t-Butylacrylamid-Terpolymere); weitere Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z. B. Polyvinylalkohol; haarkonditionierende Verbindungen (wie Phospholipide, Sojalecithin, Ei-Lecitin und Kephaline sowie Silikonöle); Proteinhydrolysate, (nsbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate); Parfümöle, Dimethylisosorbid und Cyclodextrine; faserstrukturverbessernde Wirkstoffe (wie Glucose, Galactose, Fructose, Fruchtzucker, Lactose, Maleinsäure und Milchsäure); Entschäumer wie Silikone; Farbstoffe oder Pigmente zum Anfärben des Mittels; Antischuppenwirkstoffe wie Piroctone Olamine, Zink Omadine und Climbazol; weitere Aminosäuren und Oligopeptide , insbesondere Arginin und/oder Serin; Lichtschutzmittel (derivatisierte Benzophenone, Zimtsäure-Derivate und Triazine); Wirkstoffe wie Panthenol, Pantothensäure, Pantolacton, Allantoin, Pyrrolidinoncarbonsäuren und deren Salze sowie Bisabolol; Polyphenole (insbesondere Hydroxyzimtsäuren, 6,7-Dihydroxycumarine, Hydroxybenzoesäuren, Catechine, Tannine, Leukoanthocyanidine, Anthocyanidine, Flavanone, Flavone und Flavonole); Ceramide, bevorzugt die Sphingolipide wie Ceramide I, Ceramide II, Ceramide 1, Ceramide 2, Ceramide 3, Ceramide 5 und Ceramide 6, oder Pseudoceramide, wie N-(C₈-C₂₂-Acyl)-(C₈-C₂₂-acyl)-hydroxyprolin; Vitamine, Provitamine und Vitaminvorstufen, insbesondere solche der Gruppen A, B₃, B₅, B₆, C, E, F und H; Pflanzenextrakte; Cholesterin; Konsistenzgeber wie Zuckerester, Polyolester oder Polyolalkylether; Fette und Wachse wie Bienenwachs, Montanwachs und Paraffine; Fettsäurealkanolamide; Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate; Trübungsmittel wie Latex, Styrol/PVP- und Styrol/Acrylamid-Copolymere; Perlglanzmittel wie Ethylenglykolmono- und -distearat sowie PEG-3-distearat; Treibmittel wie Propan-Butan-Gemische, N₂O, Dimethylether, CO₂ und Luft; sowie Antioxidantien.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen. Die zusätzlichen Wirk- und Hilfsstoffe werden in den erfindungsgemäßen Mitteln bevorzugt in Mengen von jeweils 0,0001 bis 10 Gew.-%, insbesondere von 0,0005 bis 5 Gew.-%, bezogen auf das Gesamtgewicht der Anwendungsmischung, eingesetzt.

In einigen Fällen hat es sich gezeigt, dass es zur Steigerung der Aufhellleistung positiv ist, wenn das erfindungsgemäße Mittel Ammoniumverbindungen enthält. Als weiteren Bestandteil können die erfindungsgemäßen Zusammensetzungen daher mindestens eine Ammoniumverbindung aus der Gruppe Ammoniumchlorid, Ammoniumcarbonat, Ammoniumbicarbonat, Ammoniumsulfat und/oder Ammoniumcarbamat enthalten. Vorzugsweise sind die Ammoniumverbindungen in einer Menge von 0,5 bis 10, vorzugsweise 1 bis 5 Gew.-%, bezogen auf die Gesamtzusammensetzung des Mittels, enthalten.

Ein weiterer Gegenstand der vorliegenden Erfindung ist ein Verfahren zum Aufhellen von Keratinfasern, insbesondere menschlichen Haaren, dadurch gekennzeichnet, dass ein Mittel des ersten Erfindungsgegenstandes auf die keratinhaltigen Fasern aufgebracht, 5 bis 60 Minuten auf der Faser belassen und anschließend wieder ausgespült oder mit einem Shampoo ausgewaschen wird. Insbesondere liegt die Temperatur während der Einwirkzeit von 5 bis 60 Minuten zwischen 10 °C und 40 °C, insbesondere zwischen 20 °C und 38 °C. Ein Aufhellverfahren, bei dem die Verbindungen der allgemeinen Struktur (I) und des Alkylethersulfats zunächst getrennt von Wasserstoffperoxid vorliegen, ist dabei bevorzugt.

Ein weiterer Gegenstand der vorliegenden Erfindung ist daher ein Verfahren zum Aufhellen von menschlichen Haaren, bei dem eine Zusammensetzung auf wässriger Grundlage, enthaltend Wasserstoffperoxid oder eine feste Anlagerungsverbindung von Wasserstoffperoxid an anorganische oder organische Verbindungen, mit einer Zusammensetzung, enthaltend mindestens ein 2-Acylpyridinium-Derivat der allgemeinen Struktur (I) und mindestens ein Alkylethersulfat zu einem Mittel der ersten Erfindungsgegenstandes vermischt, und dieses auf das Haar aufgebracht wird.

Eine weitere Ausführungsform des erfindungsgemäßen Verfahrens zum Aufhellen von menschlichen Haaren liegt vor, wenn eine Zusammensetzung auf wässriger Grundlage, enthaltend Wasserstoffperoxid oder eine feste Anlagerungsverbindung von Wasserstoffperoxid an anorganische oder organische Verbindungen, mit einem weiteren Mittel enthaltend vorzugsweise mindestens einen Alkalitätsgeber und/oder direktziehenden Haarfarbstoff und/oder mindestens ein Oxidationsfarbstoffvorprodukt, und einem Mittel enthaltend ein 2-Acylpyridinium-Derivat der allgemeinen Struktur (I) und zusätzlich einen Alkylethersulfat zu einer homogenen Zusammensetzung vermischt, und diese auf das Haar aufgebracht wird.

Um besonders starke Aufhellung zu erzielen, ist schließlich eine weitere Ausführungsform der vorliegenden Erfindung ein Verfahren, bei dem bei dem eine Zusammensetzung auf wässriger Grundlage, enthaltend Wasserstoffperoxid oder eine feste Anlagerungsverbindung von Wasserstoffperoxid an anorganische oder organische Verbindungen, mit einer Zusammensetzung, enthaltend mindestens ein 2-Acylpyridinium-Derivat der allgemeinen Struktur (I) und mindestens ein Alkylethersulfat, sowie mit einem Bleichmittel, enthaltend mindestens einen Bleichkraftverstärker, zu einem Mittel der ersten Erfindungsgegenstandes vermischt, und dieses auf das Haar aufgebracht wird. Für die Ausführungsformen des erfindungsgemäßen Verfahrens gelten die zuvor gemachten Einschränkungen sowie mutatis mutandis das zu den erfindungsgemäßen Mitteln Gesagte.

Eine bevorzugte Darreichungsform des erfindungsgemäßen Mittels ist eine Verpackungseinheit (Kit-of-Parts), welche in getrennt voneinander konfektionierten Containern
- mindestens eine Oxidationsmittelzubereitung (B), enthaltend Wasserstoffperoxid oder eine festen Anlagerungsverbindung von Wasserstoffperoxid an anorganische oder organische Verbindungen, und
- mindestens eine Zubereitung (A) enthält, wobei die Zubereitung (A) in einem kosmetischen Träger mindestens ein 2-Acylpyridinium-Derivat der allgemeinen Struktur (I) und mindestens ein Alkylethersulfat enthält.

Wird ein besonders starker Aufhelleffekt gewünscht, so ist eine bevorzugte weitere Darreichungsform des erfindungsgemäßen Mittels eine Verpackungseinheit (Kit-of-Parts), welche in getrennt voneinander konfektionierten Containern
- mindestens eine Oxidationsmittelzubereitung (B), enthaltend Wasserstoffperoxid oder eine festen Anlagerungsverbindung von Wasserstoffperoxid an anorganische oder organische Verbindungen,
- mindestens eine Blondierzubereitung (C), enthaltend mindestens einen Bleichkraftverstärker, und
- mindestens eine Zubereitung (A) enthält, wobei die Zubereitung (A) in einem kosmetischen Träger mindestens ein Acylpyridinium-Derivat der allgemeinen Struktur (I) und mindestens ein Alkylethersulfat enthält.

Bevorzugt enthält die Mehrkomponentenverpackungseinheit (Kit-of-Parts) zusätzlich eine Gebrauchsanleitung. Darüber hinaus kann es bevorzugt sein, wenn eine Applikationshilfe, wie beispielsweise ein Kamm oder ein Pinsel, und/oder eine persönliche Schutzausrüstung, wie Einweg-Handschuhe dem Kit beigefügt ist. Bezüglich weiterer bevorzugter Ausführungsformen der Mehrkomponentenverpackungseinheit (Kit-of-Parts) gilt mutatis mutandis das zu den erfindungsgemäßen Mitteln Gesagte.

Ein letzter Gegenstand der Erfindung ist die Verwendung der Mittel des ersten Erfindungsgegenstandes zum Aufhellen von keratinhaltigen Fasern, insbesondere menschlichen Haaren.

Bezüglich weiterer bevorzugter Ausführungsformen der erfindungsgemäßen Verwendung gilt mutatis mutandis das zu den erfindungsgemäßen Mitteln Gesagte.

### Beispiele

### 1.0 Synthesebeispiel

### 1.1. Synthese von 2-Acetyl-1-methylpryidinium-p-toluolsulfonat

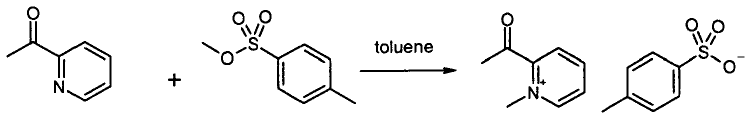

Es wurden 50,0 g (0,41 mol) 2-Acetylpyridin und 80,6 g (0,43 mol) p-Toluolsulfonsäure-methylester in 250 ml Toluol für 5 Stunden unter Rückfluss erhitzt. Nach Abkühlen wurde der entstandene hellbraune Feststoff abfiltriert, mit Toluol nachgewaschen und im Vakuum getrocknet. Ausbeute: 77,2 g (61 %); ¹H-NMR (400 MHz, DMSO-d₆): δ [ppm] = 2,30 (s, 3H); 2,81 (s, 3H); 4,35 (s, 3H); 7,10 (d, 2H); 7,47 (d, 2H); 8,26 (dd, 1H); 8,61 (d, 1H); 8,78 (dd, 1H); 9,18 (d, 1 H).

### 2. Beispiele zur Blondierung

### 2.1. Blondierungen mit Wasserstoffperoxid

### 2.1.1. Herstellung einer Blondiercreme

Aus den aufgelisteten Bestandteilen wurden Blondiercremes wie folgt hergestellt:

| **Rohstoff** | **Gew.-%** | | |
|---|---|---|---|
| | **V1** | **V2** | **E1** |
| Hydrenol D | 6,9 | 6,9 | 6,9 |
| Lorol techn. | 2,5 | 2,5 | 2,5 |
| Eumulgin B1 | 0,6 | 0,6 | 0,6 |
| Eumulgin B2 | 0,6 | 0,6 | 0,6 |
| Akypo Soft 45 NV | 10,0 | 10,0 | 10,0 |
| Plantacare 1200 UP | 2,0 | 2,0 | 2,0 |
| Texapon K 14 S 70 C | 2,8 | 2,8 | 2,8 |
| Ammoniumsulfat | 1,0 | 1,0 | 1,0 |
| Ascorbinsäure | 0,1 | 0,1 | 0,1 |
| Natriumsilikat 40 / 42 | 0,5 | 0,5 | 0,5 |
| Turpinal SL | 0,2 | 0,2 | 0,2 |
| Kaliumhydroxid | 0,8 | 0,8 | 0,8 |
| Ammoniak, 25 Gew.-% wässrig | 7,1 | 7,1 | 7,1 |
| 4-Acetyl-1-methylpyridinium-p-toluolsulfonat | - | **2,0** | - |
| 2-Acetyl-1-methylpyridinium-p-toluolsulfonat (Beispiel 1.1) | - | - | **2,0** |
| Wasser | ad 100 | ad 100 | ad 100 |

Eingesetzte Rohstoffe:
- Hydrenol^{®} D: C₁₆-C₁₈-Fettalkohol (INCI-Bezeichnung: Cetearyl alcohol) (Cognis)
- Lorol^{®} tech.: C₁₂-C₁₈-Fettalkohol (INCI-Bezeichnung: Coconut alcohol) (Cognis)
- Eumulgin^{®} B1: C₁₆-C₁₈-Fettalkohol, ethoxyliert (12 EO) (INCI-Bezeichnug: Ceteareth-12) (Cognis)
- Eumulgin^{®} B2: C₁₆-C₁₈-Fettalkohol, ethoxyliert (20 EO) (INCI-Bezeichnug: Ceteareth-20) (Cognis)
- Akypo^{®} Soft 45 NV: C₁₂-C₁₄-Fettalkoholetheressigsäure, Natriumsalz (4,5 EO) (INCI-Bezeichnung: Sodium Laureth-5 carboxylate) (KAO Chemicals)
- Plantacare^{®} 1200 UP: C₁₂-C₁₆-Fettalkoholglucoside (INCI-Bezeichnung: Lauryl Glucoside) (Cognis)
- Texapon^{®} K 14 S 70 C: Myristylethersulfat, Natriumsalz (ca. 70% Aktivsubstanz; INCI-Bezeichnung: Sodium Myreth Sulfate) (Cognis)
- Turpinal^{®} SL: 1-Hydroxyethan-1,1-diphosphonsäure (ca. 60% Aktivsubstanzgehalt; INCI-Bezeichnung: Etidronic Acid, Aqua (Water)) (Solutia)
- Natriumsilikat 40/42: Natronwasserglas

Hydrenol D, Lorol, Eumulgin B1, Eumulgin B2, Akypo Soft 45 NV, Plantacare 1200 UP und Texapon K 14 S 70 C wurden zusammen bei 80 °C aufgeschmolzen und mit einem Teil der Wassermenge dispergiert. Anschließend wurden die restlichen Rezepturbestandteile unter Rühren der Reihe nach eingearbeitet. Dann wurde mit Wasser auf 100 Gew.-% aufgefüllt und die Formulierung kalt gerührt. Bei den Rezepturen V1 und V2 handelt es sich um nicht erfindungsgemäße Vergleichsrezepturen ohne 2-Acylpyridinium-Derivat bzw. mit 4-Acylpyridinimum-Derivat, die Rezeptur E1 ist ein erfindungsgemäßes Beispiel mit dem Bleichaktivator 2-Acetyl-1-methylpyridinium-p-toluolsulfonat.

### 2.1.2. Vermischen mit der Entwicklerdispersion

Jede Blondiercreme wurde im Verhältnis 1:1 mit einer wie folgt zusammengesetzten Entwicklerdispersion ausgemischt. Der pH-Wert der Anwendungsmischung lag zwischen 9 und 10,2.

| **Rohstoff** | **Gew.-%** |
|---|---|
| Ammoniak 25 % | 0,62 |
| Dipicolinsäure | 0,10 |
| Dinatriumpyrophosphat | 0,03 |
| Turpinal SL | 1,50 |
| Texapon NSO | 2,00 |
| Dow Corning DB 110 A | 0,07 |
| Aculyn 33A | 12,00 |
| Wasserstoffperoxid, 50 Gew.-%, wässrig | 22,40 |
| Wasser | ad 100 |

- Texapon^{®} NSO: Laurylethersulfat, Natriumsalz (ca. 27,5% Aktivsubstanz; INCI-Bezeichnung: Sodium Laureth Sulfate) (Cognis)
- Aculyn^{®} 33: Acrylpolymer (ca. 28% Festkörper in Wasser; INCI-Bezeichnung: Acrylates Copolymer) (Rohm & Haas)
- Dow Corning^{®} DB 110 A: nicht ionische Silikonemulsion (INCI-Bezeichnung: Dimethicon) (Dow Corning)

Für den Blondierprozeß wurde auf Strähnen dunkelblonden (Code Kerling 7/0) und hellbraunen (Code Fischbach & Miller 6923) Haares von ca. 0,7 g Gewicht die 4-fache Menge der fertigen Anwendungsmischung appliziert. Nachdem die Strähnen für 30 Minuten bei 32 °C blondiert wurden, wurden sie mit einem handelsüblichen Shampoo gewaschen und mit einem Föhn getrocknet.

### 2.1.3. Auswertung der Aufhellleistung

Jede Haarsträhne wurde vor und nach dem Bleichvorgang farbmetrisch vermessen. Als Maß für die Aufhellleistung der jeweiligen Rezeptur wurde der dL-Wert gemäß folgender Formel herangezogen: dL = L_{nachher}- Lᵥₒᵣₕₑᵣ
L_{nachher} = Helligkeit der Strähne nach dem Bleichen
Lᵥₒᵣₕₑᵣ = Helligkeit der Strähne vor dem Bleichen

Für jede Rezeptur und jeden Haartyp erfolgte eine Zwölffachbestimmung, aus den Einzelwerten wurde jeweils der Mittelwert gebildet. Je größer der dL-Wert ist, desto besser ist die Aufhellleistung der jeweiligen Rezeptur.

Aufhellleistung bei dunkelblonden Strähnen (Kerling 7/0)

| dL (V1) | dL(V2) | dL(E1) |
|---|---|---|
| **9,5** | **12,0** | **13,6** |

Aufhellleistung bei hellbraunen Strähnen (Fischbach & Miller 6923)

| dL(V1) | dL(V2) | dL(E1) |
|---|---|---|
| **10,3** | **11,6** | **13,0** |

### 2.1.4 Deutung der Ergebnisse

Eine Abschätzung der Bleichwirkungen der unterschiedlichen Rezepturen lässt sich durch den Vergleich der dL-Werte treffen. Bei einem Vergleich der Ergebnisse wird ersichtlich, dass bei Zusatz des erfindungsgemäßem Bleichaktivators zur Blondierformulierung größere ΔL-Werte erzielt werden können und damit die Aufhelleffekte über das aus dem Stand der Technik bekannte Ausmaß hinaus vergrößert werden können.

### 2.2. Biondierungen mit Wasserstoffperoxid und Benzylalkohol bzw. Sodium Coceth-30 Sulfate

### 2.2.1. Herstellung einer Blondiercreme

Aus den aufgelisteten Bestandteilen wurden Blondiercremes wie folgt hergestellt:

| **Rohstoff** | **Gew.-%** | | | |
|---|---|---|---|---|
| | **V3** | **E2** | **E3** | **E4** |
| Hydrenol D | 6,9 | 6,9 | 6,9 | 6,9 |
| Lorol techn. | 2,5 | 2,5 | 2,5 | 2,5 |
| Eumulgin B1 | 0,6 | 0,6 | 0,6 | 0,6 |
| Eumulgin B2 | 0,6 | 0,6 | 0.6 | 0,6 |
| Akypo Soft 45 NV | 10,0 | 10,0 | 10,0 | 10,0 |
| Plantacare 1200 UP | 2,0 | 2,0 | 2,0 | 2,0 |
| Texapon K 14 S 70 C | 2,8 | 2,8 | 2,8 | 2,8 |
| Ammoniumsulfat | 1,0 | 1,0 | 1,0 | 1,0 |
| Ascorbinsäure | 0,1 | 0,1 | 0,1 | 0,1 |
| Natriumsilikat 40 / 42 | 0,5 | 0,5 | 0,5 | 0,5 |
| Turpinal SL | 0,2 | 0,2 | 0,2 | 0,2 |
| Kaliumhydroxid | 0,8 | 0,8 | 0,8 | 0,8 |
| Ammoniak, 25 Gew.-% wässrig | 7,1 | 7,1 | 7,1 | 7,1 |
| Benzylalkohol | - | - | **2,0** | - |
| Disponil FES 77 (Aktivsubstanz) | - | - | - | **2,0** |
| 2-Acetyl-1-methylpyridinium-p-toluolsulfonat (gemäß Beispiel 1.1) | - | **2,0** | **2,0** | **2,0** |
| Wasser | ad 100 | ad 100 | ad 100 | ad 100 |

| | | | | |
|---|---|---|---|---|
| Disponil^{®} FES 77 C₁₂-C₁₈-Fettalkoholethersulfat, Natriumsalz (30 EO) (ca. 31-33% Aktiv-substanz in Wasser; INCI-Bezeichnung: Sodium Coceth-30 Sulfate) (Cognis) | | | | |

Analog Beispiel 2.1 wurden Hydrenol D, Lorol, Eumulgin B1, Eumulgin B2, Akypo Soft 45 NV, Plantacare 1200 UP und Texapon K 14 S 70 C zusammen bei 80 °C aufgeschmolzen und mit einem Teil der Wassermenge dispergiert. Anschließend wurden die restlichen Rezepturbestandteile unter Rühren der Reihe nach eingearbeitet. Dann wurde mit Wasser auf 100 Gew.-% aufgefüllt und die Formulierung kalt gerührt. Bei der Rezeptur V3 handelt es sich um nicht erfindungsgemäße Vergleichsrezepturen ohne Bleichaktivator, die Rezepturen E2, E3 und E4 sind erfindungsgemäße Beispiele.

### 2.2.2. Vermischen mit der Entwicklerdispersion

Jede Blondiercreme wurde im Verhältnis 1:1 mit einer Entwicklerdispersion gemäß Beispiel 2.1 ausgemischt. Der pH-Wert der fertigen Anwendungsmischung lag zwischen 9 und 10,2. Für den Blondierprozeß wurde auf Strähnen dunkelblonden (Code Kerling 7/0), hellbraunen (Code Fischbach & Miller 6923) und dunkelbraunen (Code Kerling 2/0) Haares von ca. 0,7 g Gewicht die 4-fache Menge der fertigen Anwendungsmischung appliziert. Für alle Messungen einer Serie wurden jeweils Haare aus einer Haarcharge verwendet. Nachdem die Strähnen für 30 Minuten bei 32 °C blondiert wurden, wurden sie mit einem handelsüblichen Shampoo gewaschen und mit einem Föhn getrocknet.

### 2.2.3. Auswertung der Aufhellleistung

Für jede Rezeptur und jeden Haartyp erfolgte eine farbmetrische Zwölffachbestimmung analog Beispiel 2.1; aus den Einzelwerten wurde jeweils der Mittelwert gebildet. Je größer der dL-Wert ist, desto besser ist die Aufhellleistung der jeweiligen Rezeptur.

Aufhellleistung bei dunkelblonden Strähnen (Kerling 7/0)

| dL(V3) | dL (E2) | dL (E3) | dL (E4) |
|---|---|---|---|
| **11,47** | **13,94** | **14,48** | **14,54** |

Aufhellleistung bei hellbraunen Strähnen (Fischbach & Miller 6923)

| dL(V3) | dL (E2) | dL(E3) | dL(E4) |
|---|---|---|---|
| **10,01** | **12,10** | **14,09** | **12,34** |

Aufhellleistung bei dunkelbraunen Strähnen (Kerling 2/0)

| dL (V3) | dL(E1) | dL(E2) | dL(E3) |
|---|---|---|---|
| **4,77** | **6,87** | **8,32** | **7,10** |

### 2.1.4 Deutung der Ergebnisse

Eine Abschätzung der Bleichwirkungen der unterschiedlichen Rezepturen läßt sich durch den Vergleich der dL-Werte treffen. Es ist eindeutig ersichtlich, dass mit der erfindungsgemäßen Kombination aus Wasserstoffperoxid, einem Co-Aktivator und dem kationischen 2-Acylpyridinium-Derivat größere dL Werte und damit eine bessere Aufhellung erzielt werden können, als es durch den Einsatz des 2-Acylpyridinium-Derviates allein möglich ist.

## Patentansprüche

1. Mittel zum Aufhellen von keratinischen Fasern, **dadurch gekennzeichnet, dass** es in einem kosmetischen Träger
(i) mindestens ein kationisches 2-Acylpyridinium-Derivat der Formel (I) 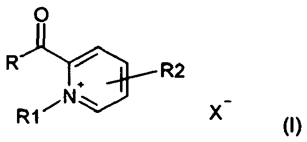 worin
R für eine C₁-C₄-Alkylgruppe, eine C₂-C₆-Hydroxyalkylgruppe oder eine C₁-C₆-Alkoxy-C₂-C₆-alkylgruppe,
R1 für eine C₁-C₆-Alkylgruppe, eine C₂-C₆-Alkenylgruppe, eine C₂-C₆-Hydroxyalkylgruppe, eine C₁-C₆-Alkoxy-C₂-C₆-alkylgruppe, eine Carboxy-C₁-C₆-alkylgruppe, eine Aryl-C₁-C₆-alkylgruppe, eine Heteroaryl-C₁-C₆-alkylgruppe, eine Mono- oder Di-(C₁-C₆-Alkyl)-Amino-C₂-C₆-Alkylgruppe, eine 3-Oxobutylgruppe, eine 2-Oxopropylgruppe, eine Arylgruppe oder eine Heteroarylgruppe steht,
R2 für Wasserstoff, ein Halogen, eine Nitrilgruppe, eine Nitrogruppe, eine Aminogruppe, eine Mono-C₁-C₆-alkylaminogruppe, eine Di-(C₁-C₆-alkyl)aminogruppe, eine Hydroxygruppe, eine C₁-C₆-Alkoxygruppe, eine C₁-C₆-Alkylgruppe, eine partiell oder vollständig halogenierte C₁-C₆-Alkylgruppe, eine C₂-C₆-Alkenylgruppe, eine C₂-C₆-Hydroxyalkylgruppe, C₁-C₆-Alkoxy-C₂-C₆-alkylgruppe, eine C₁-C₆-Alkoxycarbonylgruppe, eine Carboxygruppe, eine Carbamoylgruppe oder eine Acylgruppe R'C(O) steht, worin R' für C₁-C₄-Alkylgruppe, eine C₂-C₆-Hydroxyalkylgruppe oder eine C₁-C₆-Alkoxy-C₂-C₆-alkylgruppe steht,
X für ein physiologisch verträgliches Anion steht,
(ii) mindestens ein anionisches Tensid, ausgewählt aus Alkylethersulfaten gemäß der Formel (II) R"-(OC₂H₄)n-OSO₃M, worin R" für eine lineare oder verzweigte, gesättigte oder gegebenenfalls ungesättigte C₈-C₃₀-Alkylkette, n für eine Zahl größer als 2 und M für Wasserstoff oder ein physiologisch verträgliches Kation steht,
und
(iii) Wasserstoffperoxid oder eine feste Anlagerungsverbindung von Wasserstoffperoxid an
anorganische oder organische Verbindungen
enthält.

2. Mittel nach Anspruch 1, **dadurch gekennzeichnet, dass** R1 in der Formel (I) für eine C₁-C₆-Alkylgruppe, für eine C₂-C₆-Alkenylgruppe oder für eine C₂-C₆-Hydroxyalkylgruppe steht.

3. Mittel nach einem der Ansprüche 1 oder 2, **dadurch gekennzeichnet, dass** R2 in der Formel (I) für Wasserstoff, eine C₁-C₆-Alkylgruppe oder eine Acylgruppe R'C(O) steht, worin R' für C₁-C₄-Alkylgruppe steht.

4. Mittel nach einem der Ansprüche 1 bis 3, **dadurch gekennzeichnet, dass** mindestens eine Verbindung der Formel (I) enthalten ist, die ausgewählt wird aus mindestens einer Verbindung der Gruppe, die gebildet wird aus
2-Acetyl-1-methylpyridinium-p-toluolsulfonat,
2-Acetyl-1-methylpyridiniumbenzolsulfonat,
2-Acetyl-1-methylpyridiniumbromid,
2-Acetyl-1-methylpyridiniumchlorid,
2-Acetyl-1-methylpyridiniumhydrogensulfat,
2-Acetyl-1-methylpyridiniumacetat,
2,6-Diacetyl-1-methylpyridinium-p-toluolsulfonat, 2,6-Diacetyl-1-methylpyridiniumbenzolsulfonat,
2,6-Diacetyl-1-methylpyridiniumbromid,
2,6-Diacetyl-1-methylpyridiniumchlorid,
2,6-Diacetyl-1-methylpyridiniumhydrogensulfat und
2,6-Diacetyl-1-methylpyridiniumacetat.

5. Mittel nach einem der Ansprüche 1 bis 4, **dadurch gekennzeichnet, dass** die 2-Acylpyridinium-Derivate der Formel (I) in einer Menge von 0,01 bis 25 Gew.-%, insbesondere von 0,1 bis 10 Gew.-%, jeweils bezogen das Gesamtgewicht des anwendungsbereiten Mittels, enthalten sind.

6. Mittel nach einem der Ansprüche 1 bis 5, **dadurch gekennzeichnet, dass** das Akylethersulfat für eine Verbindung gemäß der Formel R"-(OCH₂CH₂)ₙ-OSO₃M steht, worin R" für eine gegebenenfalls verzweigte C₈-C₂₂-Alkylgruppe und n für eine Zahl von 16 bis 50, insbesondere von 20 bis 40, steht.

7. Mittel nach einem der Ansprüche 1 bis 5, **dadurch gekennzeichnet, dass** das Akylethersulfat für eine Verbindung gemäß der Formel R"-(OCH₂CH₂)ₙ-OSO₃M steht, worin R" für eine C₁₀-C₁₈-Alkylgruppe und n für eine Zahl von 25 bis 35, steht.

8. Mittel nach einem der Ansprüche 1 bis 7, **dadurch gekennzeichnet, dass** es weiterhin mindestens ein aromatisches, organisches Lösungsmittel enthält.

9. Mittel nach einem der Ansprüche 1 bis 8, **dadurch gekennzeichnet, dass** es Benzylalkohol enthält.

10. Mittel nach einem der Ansprüche 1 bis 9, **dadurch gekennzeichnet, dass** Glycin und/oder dessen physiologisch verträgliches Salz enthalten ist.

11. Mittel nach einem der Ansprüche 1 bis 10, **dadurch gekennzeichnet, dass** es einen pH-Wert von 7 bis 11 besitzt.

12. Mittel nach einem der Ansprüche 1 bis 11, **dadurch gekennzeichnet, dass** zusätzlich mindestens ein anorganisches Persulfat- oder Peroxodisulfat-Salz, insbesondere Ammoniumperoxodisulfat, Kaliumperoxodisulfat und/oder Natriumperoxodisulfat, enthalten ist.

13. Verfahren zum Aufhellen von keratinischen Fasern, **dadurch gekennzeichnet, dass** ein Mittel nach einem der Ansprüche 1 bis 12 auf die keratinhaltigen Fasern aufgebracht, 5 bis 60 Minuten auf der Faser belassen und anschließend wieder ausgespült oder mit einem Shampoo ausgewaschen wird.

14. Kosmetische Verwendung eines Mittels nach einem der Ansprüche 1 bis 12 zum Aufhellen von keratinhalten Fasern, insbesondere menschlichen Haaren.

15. Kit-of-Parts zum Aufhellen keratinischer Fasern, **dadurch gekennzeichnet, dass** es in getrennt voneinander konfektionierten Containern mindestens eine Oxidationsmittelzubereitung (B), enthaltend Wasserstoffperoxid oder eine festen Anlagerungsverbindung von Wasserstoffperoxid an anorganische oder organische Verbindungen, und mindestens eine Zubereitung (A) enthält, wobei die Zubereitung (A) in einem kosmetischen Träger mindestens ein 2-Acylpyridinium-Derivat der allgemeinen Struktur (I) gemäß Anspruch 1 und mindestens ein Alkylethersulfat enthält.

## Claims

1. An agent for lightening keratinous fibers, **characterized in that** it contains in a cosmetic carrier
(i) at least one cationic 2-acylpyridinium derivative of formula (I) 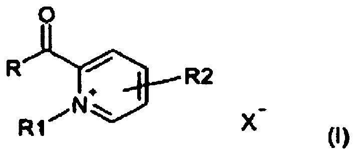 wherein
R represents a C₁-C₄ alkyl group, a C₂-C₆ hydroxyalkyl group or a (C₁-C₆ alkoxy)-(C₂-C₆ alkyl) group,
R1 represents a C₁-C₄ alkyl group, a C₂-C₆ alkenyl group, a C₂-C₆ hydroxyalkyl group, a (C₁-C₆ alkoxy)-(C₂-C₆ alkyl) group, a carboxy-(C₁-C₆ alkyl) group, an aryl-(C₁-C₆ alkyl) group, a heteroaryl-(C₁-C₆ alkyl) group, a mono-or di-(C₁-C₆ alkyl)-amino-(C₂-C₆ alkyl) group, a 3-oxobutyl group, a 2-oxopropyl group, an aryl group or a heteroaryl group,
R2 represents a hydrogen, a halogen, a nitrile group, a nitro group, an amino group, a mono-(C₁-C₆ alkyl)amino group, a di-(C₁-C₆ alkyl)amino group, a hydroxyl group, a C₁-C₆ alkoxy group, a C₁-C₆ alkyl group, a partially or totally halogenated C₁-C₆ alkyl group, a C₂-C₆ alkenyl group, a C₂-C₆ hydroxyalkyl group, a (C₁-C₆ alkoxy)-(C₂-C₆ alkyl) group, a (C₁-C₆ alkoxy)carbonyl group, a carboxy group, a carbamoyl group or an acyl group R'C(O), wherein R' represents C₁-C₄ alkyl group, a C₂-C₆ hydroxyalkyl group or a (C₁-C₆ alkoxy)-(C₂-C₆ alkyl) group,
X represents a physiologically acceptable anion,
(ii) at least one anionic surfactant, selected from alkyl ether sulfates according to formula (II) R"-(OC₂H₄)ₙ-OSO₃M, wherein R" represents a linear or branched, saturated or optionally unsaturated C₈-C₃₀ alkyl chain, n represents a number greater than 2 and M represents a hydrogen or a physiologically acceptable cation,
and
(iii) hydrogen peroxide or a solid addition compound of hydrogen peroxide on inorganic or organic compounds.

2. The agent according to claim 1, **characterized in that** R1 in formula (I) represents a C₁-C₆ alkyl group, a C₂-C₆ alkenyl group, or a C₂-C₆ hydroxyalkyl group.

3. The agent according to one of claims 1 or 2, **characterized in that** R2 in formula (I) represents a hydrogen, a C₁-C₆ alkyl group or an acyl group R'C(O), wherein R' represents C₁-C₄ alkyl group.

4. The agent according to one of claims 1 to 3, **characterized in that** at least one compound of formula (I) is contained, which is selected from at least one compound of the group which is formed of 2-acetyl-1-methylpyridinium p-toluene-sulfonate, 2-acetyl-1-methylpyridinium benzene-sulfonate, 2-acetyl-1-methylpyridinium bromide,
2-acetyl-1-methylpyridinium chloride,
2-acetyl-1-methylpyridinium hydrogensulfate, 2-acetyl-1-methylpyridinium acetate,
2,6-diacetyl-1-methylpyridinium p-toluene-sulfonate, 2,6-diacetyl-1-methylpyridinium benzene-sulfonate, 2,6-diacetyl-1-methylpyridinium bromide, 2,6-diacetyl-1-methylpyridinium chloride, 2,6-diacetyl-1-methylpyridinium hydrogensulfate and
2,6-diacetyl-1-methylpyridinium acetate.

5. The agent according to one of claims 1 to 4, **characterized in that** the 2-acylpyridinium derivatives of formula (I) are contained in an amount from 0.01 to 25% by weight, in particular from 0.1 to 10% by weight, each based on the total weight of the ready-to-use agent.

6. The agent according to one of claims 1 to 5, **characterized in that** the alkyl ether sulfate represents a compound according to formula R"-(OCH₂CH₂)ₙ-OSO₃M, wherein R" represents an optionally branched C₈-C₂₂ alkyl group and n represents a number from 16 to 50, in particular, from 20 to 40.

7. The agent according to one of claims 1 to 5, **characterized in that** the alkyl ether sulfate represents a compound according to formula R"-(OCH₂CH₂)ₙ-OSO₃M, wherein R" represents C₁₀-C₁₈ alkyl group and n represents a number from 25 to 35.

8. The agent according to one of claims 1 to 7, **characterized in that** it further contains at least one aromatic, organic solvent.

9. The agent according to one of claims 1 to 8, **characterized in that** it contains benzyl alcohol.

10. The agent according to one of claims 1 to 9, **characterized in that** glycine and/or its physiologically acceptable salt is contained.

11. The agent according to one of claims 1 to 10, **characterized in that** it has a pH value from 7 to 11.

12. The agent according to one of claims 1 to 11, **characterized in that** additionally at least one inorganic persulfate or peroxodisulfate salt, in particular, ammonium peroxodisulfate, potassium peroxodisulfate and/or sodium peroxodisulfate.

13. A method for lightening keratinous fibers, **characterized in that** an agent according to one of claims 1 to 12 is applied on the keratinous fibers, left for 5 to 60 minutes on the fiber and subsequently rinsed off again or washed off with a shampoo.

14. Cosmetic use of an agent according to one of claims 1 to 12, for lightening keratinous fibers, in particular human hair.

15. A kit of parts for lightening keratinous fibers, **characterized in that** it contains in containers made separate from each other, at least one oxidation agent preparation (B), containing hydrogen peroxide or a solid addition compound of hydrogen peroxide on inorganic or organic compounds, and at least one preparation (A), the preparation (A) containing in a cosmetic carrier, at least one 2-acylpyridinium derivative of the general structure (I) according to claim 1, and at least one alkyl ether sulfate.

## Revendications

1. Agent pour l'éclaircissement de fibres kératiniques, **caractérisé en ce qu'**il contient, dans un support cosmétique :
(i) au moins un dérivé cationique de 2-acylpyridinium répondant à la formule (I) 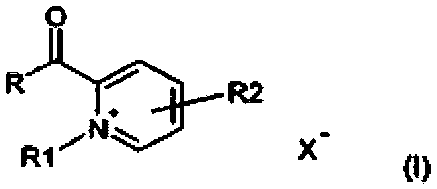 dans laquelle
R représente un groupe alkyle en C₁-C₄, un groupe hydroxyalkyle en C₂-C₆ ou un groupe alcoxy(en C₁-C₆)-alkyle en C₂-C₆ ;
R1 représente un groupe alkyle en C₁-C₆, un groupe alcényle en C₂-C₆, un groupe hydroxyalkyle en C₂-C₆, un groupe alcoxy(en C₁-C₆)-alkyle en C₂-C₆, un groupe carboxyalkyle en C₁-C₆, un groupe arylalkyle en C₁-C₆, un groupe hétéroarylalkyle en C₁-C₆, un groupe mono- ou dialkyl(en C₁-C₆)-aminoalkyle en C₂-C₆, un groupe 3-oxobutyle, un groupe 2-oxopropyle, un groupe aryle ou un groupe hétéroaryle ;
R2 représente un atome d'hydrogène, un atome d'halogène, un groupe nitrile, un groupe nitro, un groupe amino, un groupe monoalkyl(en C₁-C₆)amino, un groupe dialkyl(en C₁-C₆)amino, un groupe hydroxyle, un groupe alcoxy en C₁-C₆, un groupe alkyle en C₁-C₆, un groupe alkyle en C₁-C₆ partiellement ou complètement halogéné, un groupe alcényle en C₂-C₆, un groupe hydroxyalkyle en C₂-C₆, un groupe alcoxy(en C₁-C₆)-alkyle en C₂-C₆, un groupe alcoxy(en C₁-C₆)carbonyle, un groupe carboxyle, un groupe carbamoyle ou un groupe acyle R'C(O), R' représentant un groupe alkyle en C₁-C₄, un groupe hydroxyalkyle en C₂-C₆ ou un groupe alcoxy(en C₁-C₆)-alkyle en C₂-C₆,
X⁻ représente un anion physiologiquement acceptable ;
(ii) au moins un agent tensioactif anionique choisi parmi des alkyléthersulfates répondant à la formule (II) R"-(OC₂H₄)ₙ-OSO₃M où R" représente une chaîne alkyle en C₈-C₃₀ linéaire ou ramifiée, saturée ou éventuellement insaturée, n représente un nombre supérieur à 2 et M représente un atome d'hydrogène ou un cation physiologiquement acceptable ; et
(iii) du peroxyde d'hydrogène ou un composé solide de fixation par addition de peroxyde d'hydrogène sur des composés inorganiques ou organiques.

2. Agent selon la revendication 1, **caractérisé en ce que** R1 dans la formule (I) représente un groupe alkyle en C₁-C₆, un groupe alcényle en C₂-C₆ ou un groupe hydroxyalkyle en C₂-C₆.

3. Agent selon la revendication 1 ou 2, **caractérisé en ce que** R2 dans la formule (I) représente un atome d'hydrogène, un groupe alkyle en C₁-C₆ ou un groupe acyle R'C(O), R' représentant un groupe alkyle en C₁-C₄.

4. Agent selon l'une quelconque des revendications 1 à 3, **caractérisé en ce qu'**il contient au moins un composé répondant à la formule (I) qui est choisi parmi au moins un composé du groupe qui est formé à partir du p-toluènesulfonate de 2-acétyl-1-méthylpyridinium,
du benzènesulfonate de 2-acétyl-1-méthylpyridinium,
du bromure de 2-acétyl-1-méthylpyridinium,
du chlorure de 2-acétyl-1-méthylpyridinium,
de l'hydrogénosulfate de 2-acétyl-1-méthylpyridinium,
de l'acétate de 2-acétyl-1-méthylpyridinium,
du p-toluènesulfonate de 2,6-diacétyl-1-méthylpyridinium,
du benzènesulfonate de 2,6-diacétyl-1-méthylpyridinium,
du bromure de 2,6-diacétyl-1-méthylpyridinium,
du chlorure de 2,6-diacétyl-1-méthylpyridinium,
de l'hydrogénosulfate de 2,6-diacétyl-1-méthylpyridinium, et
de l'acétate de 2,6-diacétyl-1-méthylpyridinium.

5. Agent selon l'une quelconque des revendications 1 à 4, **caractérisé en ce qu'**il contient les dérivés de 2-acylpyridinium répondant à la formule (I) en une quantité de 0,01 à 25 % en poids, en particulier de 0,1 à 10 % en poids, chaque fois rapportés au poids total de l'agent prêt à l'emploi.

6. Agent selon l'une quelconque des revendications 1 à 5, **caractérisé en ce que** l'alkyléthersulfate représente un composé répondant à la formule R"-(OCH₂CH₂)ₙ-OSO₃M où R" représente un groupe alkyle en C₈-C₂₂ facultativement ramifié et n représente un nombre de 16 à 50, en particulier de 20 à 40.

7. Agent selon l'une quelconque des revendications 1 à 5, **caractérisé en ce que** l'alkyléthersulfate représente un composé répondant à la formule R"-(OCH₂CH₂)ₙ-OSO₃M où R" représente un groupe alkyle en C₁₀-C₁₈ et n représente un nombre de 25 à 35.

8. Agent selon l'une quelconque des revendications 1 à 7, **caractérisé en ce qu'**il contient en outre au moins un solvant organique aromatique.

9. Agent selon l'une quelconque des revendications 1 à 8, **caractérisé en ce qu'**il contient de l'alcool benzylique.

10. Agent selon l'une quelconque des revendications 1 à 9, **caractérisé en ce qu'**il contient de la glycine et/ou son sel physiologiquement acceptable.

11. Agent selon l'une quelconque des revendications 1 à 10, **caractérisé en ce qu'**il possède une valeur de pH de 7 à 11.

12. Agent selon l'une quelconque des revendications 1 à 11, **caractérisé en ce qu'**il contient en outre au moins un sel inorganique de persulfate ou de peroxodisulfate, en particulier du peroxodisulfate d'ammonium, du peroxodisulfate de potassium et/ou du peroxodisulfate de sodium.

13. Procédé pour l'éclaircissement de fibres kératiniques, **caractérisé en ce qu'**on applique un agent selon l'une quelconque des revendications 1 à 12 sur les fibres kératiniques, on le laisse sur les fibres pendant un laps de temps de 5 à 60 minutes et on l'en élimine ensuite par rinçage ou par lavage avec un shampooing.

14. Utilisation cosmétique d'un agent selon l'une quelconque des revendications 1 à 12, pour l'éclaircissement de fibres kératiniques, en particulier de cheveux humains.

15. « Kit-of-parts » pour l'éclaircissement de fibres kératiniques, **caractérisé en ce qu'**il contient, dans des récipients confectionnés pour être séparés les uns des autres, au moins une préparation d'agent d'oxydation (B) contenant du peroxyde d'hydrogène ou bien un composé solide de fixation par addition du peroxyde d'hydrogène à des composés inorganiques ou organiques, et au moins une préparation (A), la préparation (A) contenant, dans un support cosmétique, au moins un dérivé de 2-acylpyridinium répondant à la formule générale (I) selon la revendication 1, et au moins un alkyléthersulfate.
